# EUROPEAN PATENT SPECIFICATION

(11) **EP 2 704 697 B1**
(45) Date of publication and mention of the grant of the patent: **12.09.2018**
(21) Application number: 12782731.9
(22) Date of filing: 04.05.2012
(51) Int. Cl.: A61K 9/24, A61K 31/167, A61P 29/02

(54) **SUSTAINED RELEASE PARACETAMOL FORMULATIONS**
PARACETAMOLFORMULIERUNGEN MIT VERZÖGERTER FREISETZUNG
FORMULATIONS DE PARACÉTAMOL À LIBÉRATION PROLONGÉE

(30) Priority: 06.05.2011 US 201161483320 P
(43) Date of publication of application: 12.03.2014
(73) Proprietor: GlaxoSmithKline Consumer Healthcare (UK) IP Limited, Brentford, Middlesex TW8 9GS (GB)
(72) Inventor: BUAN, Carla, Valenti, Parsippany NJ 07054 (US); LIU, Dongzhou, Parsippany NJ 07054 (US); MEGHPARA, Kanji, Parsippany NJ 07054 (US)
(74) Representative: Morris, Miriam Elizabeth
(86) International application number: PCT/US2012/036528
(87) International publication number: WO 2012/154563

(56) References cited:
- WO-A1-97/39747
- WO-A1-2009/100118
- WO-A2-2008/131056
- WO-A2-2011/026125
- US-A- 5 055 306
- US-A- 5 849 240
- US-A1- 2003 064 108
- US-A1- 2004 170 681
- US-A1- 2004 202 716
- US-A1- 2005 158 382
- US-A1- 2005 175 706
- US-A1- 2006 127 484
- US-A1- 2008 200 549
- US-A1- 2010 104 638
- US-B1- 6 599 529
- STRÖM C ET AL: "Analgesic efficacy of acetaminophen sustained release", JOURNAL OF CLINICAL PHARMACOLOGY, vol. 30, no. 7, July 1990 (1990-07), pages 654-659, XP002732247,
- SKOGLUND L A ET AL: "Comparison of a traditional paracetamol medication and a new paracetamol/paracetamol-methionine ester combination", EUR J CLIN PHARMACOL, vol. 26, no. 5, 1984, pages 573-577, XP008173192,
- BOURNE T H ET AL: "Paracetamol-associated luteinized unruptured follicle syndrome: effect on intrafollicular blood flow", ULTRASOUND IN OBSTETRICS & GYNECOLOGY : THE OFFICIAL JOURNAL OF THE INTERNATIONAL SOCIETY OF ULTRASOUND IN OBSTETRICS AND GYNECOLOGY, vol. 1, no. 6, 1 November 1991 (1991-11-01), pages 420-425, XP002732248,
- GREENBERG R N: "Overview of patient compliance with medication dosing: A literature review", CLINICAL THERAPEUTICS, EXCERPTA MEDICA, PRINCETON, NJ, US, vol. 6, no. 5, 1 January 1984 (1984-01-01) , pages 592-599, XP008173120, ISSN: 0149-2918

## Description

### FIELD OF THE INVENTION

The present invention is directed to twice daily sustained release pharmaceutical compositions of paracetamol having an immediate release phase and a sustained release phase of paracetamol and having unique and advantageous pharmacokinetic properties.

### BACKGROUND OF THE INVENTION

The present invention relates to pharmaceutical compositions containing N-acetyl-p-aminophenol, known by the generic names paracetamol, acetaminophen and APAP (hereinafter referred to as paracetamol). In particular, the invention relates to a sustained release paracetamol formulation having an advantageous pharmacokinetic profile.

Paracetamol is an analgesic and antipyretic agent widely used in prescription and non-prescription medicines, often in combination with other biologically active compounds, such as various opiate derivatives.

The elimination half-life of paracetamol is reported to be in the range of 1.9-2.5 hours.
Its absorption following oral doses of conventional immediate release tablets is characterized by passive absorption with high bioavailability (80%) and rapidly occurring maximum plasma concentration (tₘₐₓ 30-90 min). These characteristics determine a conventional dosage regimen of 1000mg paracetamol being administered every 4 to 6 hours. Although this regimen is acceptable for the short-term treatment of acute pain, it becomes inconvenient for long-term treatment of sub-chronic or chronic pain.
Osteoarthritis (OA) and musculoskeletal disorders prevalence is widely associated with ageing. Globally data is available which demonstrates that while 25% of all pain occasions are headache, joint pain represents 14% of all pain occasions. This percentage will likely increase proportionally with increasing age and changes dramatically in people over 60 to be 55% of all pain occasions.

In joint pain, generally a chronic condition, medication compliance is an essential component for achieving optimal efficacy. Issues of compliance are particularly important for elderly patients who can have a range of co-morbidities requiring pharmacological treatment. Sustained release paracetamol formulations can improve a patient's quality of life by reducing the number of doses to be taken, and providing steadier levels of the drug in the blood as determined by plasma or serum drug concentrations.

Paracetamol is recommended as first-line treatment because it effectively relieves the mild to moderate pain of OA, while avoiding non-steroidal anti-inflammatory drug (NSAID)-associated risks, such as gastrointestinal (GI), cardiovascular and renal complications and has few drug interactions. Even at OTC doses, NSAIDs such as ibuprofen and aspirin have the potential to produce significant adverse GI effects when used regularly. Elderly patients are also at a greater risk from serious GI events.
A paracetamol product designed for three times daily dosing (tid) will contain enough paracetamol to give close to the maximum daily dose when two tablets are taken three times daily, i.e., about 600 mg to 667 mg per tablet. Such a product is sold as Panadol® Extend around the world and is described in US 7,943,170. The product described in US 7,943,170 is a sustained release bilayer tablet containing 665 mg of paracetamol. These tablets contain 70% of paracetamol in a sustained release layer and 30% in an immediate release layer. The sustained release layer in these tablets is provided by a matrix comprising a mixture of hydroxypropylmethylcellulose and polyvinyl-pyrrolidone.
The product described in EP-A-305051 (McNeil, Inc) is a sustained release bilayer tablet containing either 650 or 667 mg of paracetamol. These tablets contain equal amounts of paracetamol in an immediate release layer and a sustained release layer. The sustained release layer in these tablets is provided by a matrix comprising a mixture of hydroxyethylcellulose and polyvinyl-pyrrolidone. McNeil, Inc. markets such a bilayer tablet as Tylenol® Extended Relief in the US.
The product described in US 5,773,031, Shah et al. is a mixture of polymeric coated, sustained release acetaminophen particles and uncoated, quick release acetaminophen particles pressed together in a tablet. The coating is water permeable, but not soluble or pH dependent, e.g., a water-insoluble, pH independent coating. The patent does not describe a release rate profile for the acetaminophen.
There are many additional publications on sustained release formulations of paracetamol; however none of the above appear to have addressed the issue of twice daily dosing of the active agent. While three times daily dosing has significant advantages over every 4 hours or every 6 hours, twice daily dosing is preferred for not only patient compliance, but to be able to maintain a therapeutic effectiveness over longer periods of time for analgesic control. Attaining suitable pharmacokinetic profiles at twice daily dosing has been found to be extremely difficult. Many years of experimentation has failed to achieve the desired concentration levels over the necessary time periods.

US 2005/158382 A1 discloses triple layer tablets comprising an immediate release layer of paracetamol and hydrocodone bitartrate, a sustained release layer of paracetamol and a sustained release layer of hydrocodone bitartrate. The tablets are administrable on a twice-a-day basis. There is a need in the art for a formulation for twice daily administration, e.g., a 12 hour dosing regimen of paracetamol, which can achieve and maintain a therapeutic effectiveness for up to 12 hours, suitably at steady state.

### Brief Description of the Drawings

Figure 1a shows the biorelevant dissolution profiles for the product of Example 1, a conventional immediate release paracetamol formulation, and an 8-hour extended release formulation, Panadol Extend®.
Figure 1b shows the biorelevant dissolution profiles for Examples 2 through 6b. Examples 2 to 5c are not according to the invention.

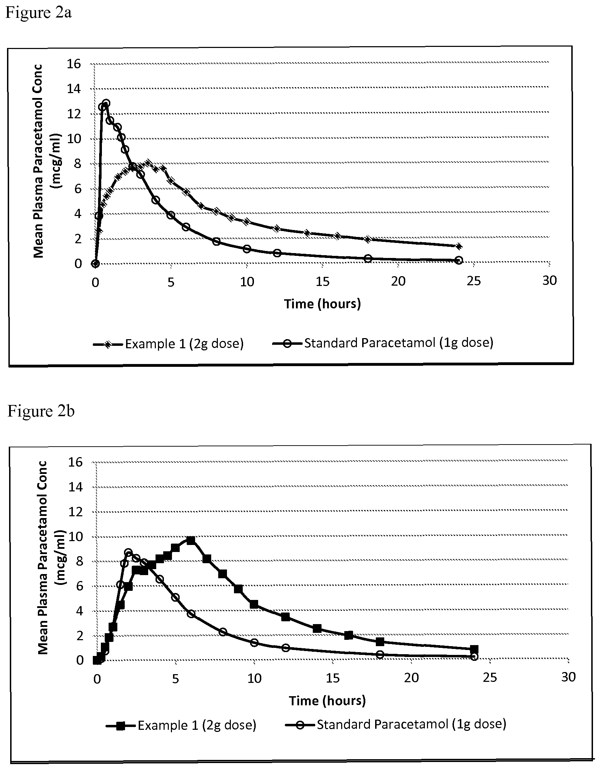

Figure 2a shows single dose pharmacokinetic profiles in the fasted state for a 2000mg dose of the product of Example 1, and a 1000mg dose of a conventional immediate release paracetamol formulation.
Figure 2b shows single dose pharmacokinetic profiles in the fed state for a 2000mg dose of the product of Example 1, and a 1000mg dose of a conventional immediate release paracetamol formulation.

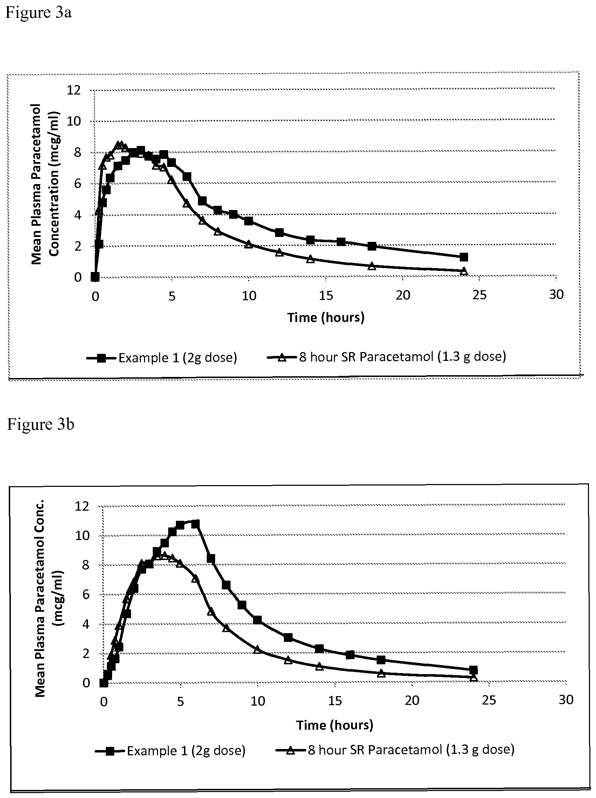

Figure 3a shows single dose pharmacokinetic profiles in the fasted state for a 2000mg dose of the product of Example 1 and for a 1330mg dose of an 8-hour extended release formulation, Panadol Extend®.
Figure 3b shows single dose pharmacokinetic profiles in the fed state for a 2000mg dose of the product of Example 1, and for a 1330mg dose of an 8-hour extended release formulation, Panadol Extend®.
Figure 4 shows multiple dose (Steady-State) pharmacokinetic profiles over a 24 hour period for a 2000mg dose of Example 1 given every 12 hours, and for a 1000mg dose of a conventional immediate release paracetamol formulation given every 6 hours, and for a 1330mg dose of an 8-hour extended release formulation, Panadol Extend® given every 8 hours (*based on low absorption in the colon beyond 6 hours).
Figure 5 shows the predicted therapeutic effective times for Examples 1 through 6 based upon IVMS. TET is the time from when the medicine starts to work and becomes effective, to the time the effect of the medicine is gone. Examples 2 to 5c are not according to the invention.

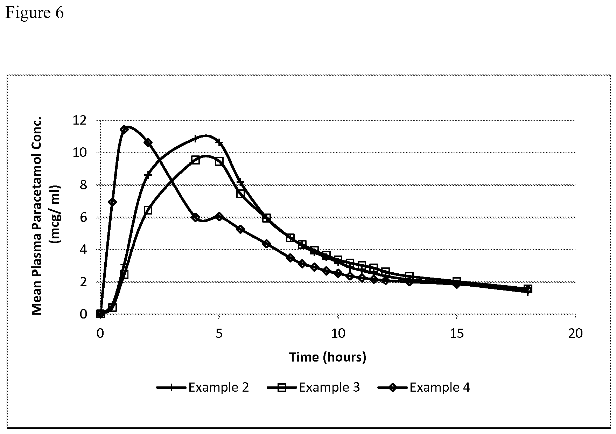

Figure 6 shows a single dose pharmacokinetic profile in a semi-fed state for the product of Example 2, Example 3 and Example 4. Examples 2-4 are not according to the invention.

### SUMMARY OF THE INVENTION

The present invention is directed to a sustained release formulation for oral administration containing 2000mg of paracetamol per unit dose administered as two bilayer tablets, each tablet containing 1000mg of paracetamol present in a sustained release phase and in an immediate release phase, in which the ratio of the paracetamol in the sustained release phase to the immediate release phase is 80-90% to 10-20%, and wherein the sustained release phase comprises 4% to 6% by weight of at least one high viscosity hypromellose, 0.5% to 3% by weight of at least one binding agent which is povidone, from 1% to 2% by weight of a low viscosity hypromellose, 0.5% to 3% by weight of at least one modified starch, and wherein the ratio of high-viscosity hypromellose to low viscosity hypromellose is 3.3 to 0.85, wherein when ingested by a human, the formulation reduces the maximum attained plasma-paracetamol concentration by at least about 4.5% at steady state (relative to rapid-release paracetamol formulations'), and increases the time to reach a maximum paracetamol-plasma concentration (T*ₘₐₓ*) by at least about 140% at steady state (relative to rapid-release paracetamol formulations'), while having an insignificant effect on area under the plasma-paracetamol concentration time curve AUC₍₀₋₂₄₎; mean AUC₍₀₋₂₄₎ of about 165 µg*h/ml for sustained release paracetamol at steady state (2000mg dosed every 12 hours) versus a mean AUC₍₀₋₂₄₎ of about 168 µg*h/ml for 1000mg immediate release at steady state (dosed every 6 hours) and wherein the formulation is repeatedly administered (steady state).

In another embodiment, the invention is directed to the above sustained release formulation for oral administration providing a therapeutically effective plasma paracetamol concentration over a 12 hour period for use in the treatment of pain in a human in need thereof wherein the formulation has the following in vitro bio-dissolution profile of the dissolution release ranges at various time points (as determined by USP Type II apparatus, rotating paddle, with 900 ml of Phosphate buffer at pH 7.4, 37 °C set at rotating speed of 75rpm) of:
2 to 15% released at 15 minutes;
4 to 22% released at 30 minutes;
10 to 40% released at 60 minutes;
22 to 62% released at 180 minutes;
50 to 88% released at 360 minutes;
>90% released after 720 minutes.

### DETAILED DESCRIPTION OF THE INVENTION

### Definitions

As used herein, the term "PK (pharmacokinetics)" refers to the study of the absorption, distribution, metabolism, and excretion of drugs.

As used herein, the term "PD (pharmacodynamics)" refers to the relationship between drug concentration and pharmacological response.

As used herein, the term "steady state" means that the blood plasma concentration curve for a given drug does not substantially fluctuate after repeated doses to dose of the formulation.

As used herein, the term "dosage form" refers to at least one dosage unit form of the present invention, that is one tablet containing 1000mg each of paracetamol (in the present invention, each single dose of paracetamol is contained in 2 unit dosage forms of the sustained release formulation herein for twice-a-day administration, for a total daily intake of 4000mg).

As used herein, the terms "single dose"," unit dosage" or "unit dose", used interchangeably, mean that the human patient has received a single dose of the drug formulation and the drug plasma concentration has not achieved a steady state. For the present invention, a single dose, unit dosage or unit dose consists of two tablets, each tablet containing 1000mg of sustained release paracetamol for a total of 2000mg per single dose, unit dosage or unit dose.

As used herein, the term "multiple dose" means that the human patient has received at least two doses of the drug formulation in accordance with the dosing interval for that formulation (e.g., on a twice-a-day basis). Patients who have received multiple doses of the sustained release formulation of the invention may or may not have attained steady state drug plasma levels, as the term multiple dose is defined herein.

As used herein, the term "mean", when preceding a pharmacokinetic value represents the arithmetic mean value of the pharmacokinetic value taken from a population of patients unless otherwise specified (e.g., geometric mean).

As used herein, the term "Cₘₐₓ" refers to the maximum plasma concentration.

As used herein, the term "C*ₘᵢₙ*" refers to the minimum plasma concentration reached after a drug has been dosed and prior to the administration of a second dose.

As used herein, the term "T*ₘₐₓ*" refers to the time to reach maximum plasma concentration.

As used herein the term "Kₑₗ" (hour) refer to the elimination rate constant. It is the terminal slope (using an In C versus time plot) of the serum concentration/time curve after absorption and distribution phases are complete. The half-life and elimination rate constant are related to each other by the following equation: T_{1/2} = 0.693/Kₑₗ.
As used herein the term T_{lag} refers to the time delay prior to the start of drug absorption. This may be due to physiologic factors such as stomach emptying time and intestinal mobility.

As used herein "T_{1/2}" (hour) refers to the half life or half time elimination. It is the time required for serum concentrations to decrease by one-half after absorption and distribution are complete. See DiPiro JT, Talbert RL, Yee GC, et al: Pharmacotherapy: A Pathophysiologic Approach, 7e.

As used herein, the term "bioavailability" means the rate and extent to which the active drug substance is absorbed from a pharmaceutical dosage form and becomes available at the site of action.

As used herein, the term "bioequivalence" (BE) is the absence of a significant difference in the rate and extent to which the active ingredient becomes available at the site of drug action when administered at the same molar dose under similar conditions in an appropriately designed study. In the context of a generic containing the same active ingredient in the same amount, is considered to be bioequivalent to a brand/reference and/or listed drug product if the rate and extent of absorption do not show a significant difference from the listed drug, or the extent of absorption does not show a significant difference and any difference in rate is intentional or not medically significant.

As used herein, the term "generic drug" means a drug product that is comparable to a brand/reference listed drug product in dosage form, strength, route of administration, quality and performance characteristics and intended use.

The present invention is directed to a sustained release formulation of paracetamol that provides efficacy over a period of at least 10 hours or more, or alternatively at least 12 hours, and can therefore be optionally dosed twice daily. The present invention is also directed to an optionally twice daily administration of a unit dosage sustained release formulation of paracetamol that has a lower Cₘₐₓ in the fasted state, and has a slower rate of absorption as compared to a conventional immediate release formulation of paracetamol (of a similar daily dose). The present invention is also directed to an optionally twice daily administration of a sustained release formulation of paracetamol that provides an equivalent time duration of plasma paracetamol concentration at or above therapeutic level, i.e., greater than or equal to 3 - 5µg/ml. Suitably, the duration of action is over a period of at least 10 hours, preferably longer, e.g., up to 11 or 12 hours. The present invention is also directed to an optionally twice daily administration of a unit dosage sustained release formulation of paracetamol which provides for a lower fluctuation index than a conventional immediate release formulation of a similar amount of paracetamol administered over the course of the day.

In one embodiment the present invention is an optionally twice daily administration of a unit dosage sustained release formulation of paracetamol that is well absorbed in both a fed and fasted state. The formulation provides for more than 90% relative bioavailability, as compared to a conventional immediate release formulation and as compared to an 8-hour extended release formulation on a dose adjusted basis.

In one embodiment the present invention is an optionally twice daily administered unit dosage sustained release formulation of paracetamol that provides certain pharmacokinetic parameters, such as a lower Kₑₗ (elimination rate) and a longer half - time elimination (T ½) that are significantly different when compared to a conventional immediate release formulation and when compared to an 8-hour extended release formulation in a single dose study.

In one embodiment the present invention is an optionally twice daily administered unit dosage sustained release formulation of paracetamol that provides a median time to reach a plasma paracetamol concentration of up to 4µg/ml that is similar to a conventional immediate release formulation. The amount of paracetamol in the immediate release layer of the present invention available to the subject in a clinical study was only 1/5th the dose (200mg) available in the conventional formulation (e.g., a 1000mg (2x 500mg tablet dose).

In one embodiment the present invention is an optionally twice daily administered unit dosage of 2000mg paracetamol in a sustained release formulation of paracetamol that delivers 1000mg paracetamol per tablet in a two (2) tablet administration wherein each tablet is an easy to swallow condensed tablet dosage form weighing only about 1110mg thus providing improved patient compliance.

In one embodiment the present invention is an optionally twice daily administered unit dosage sustained release formulation of paracetamol that provides a biorelevant dissolution profile that is significantly longer, e.g., 12 hours as opposed to 5 hours for an 8-hour extended release product, i.e., Panadol Extend®, or 30 minutes for a conventional immediate release dosage form of paracetamol.

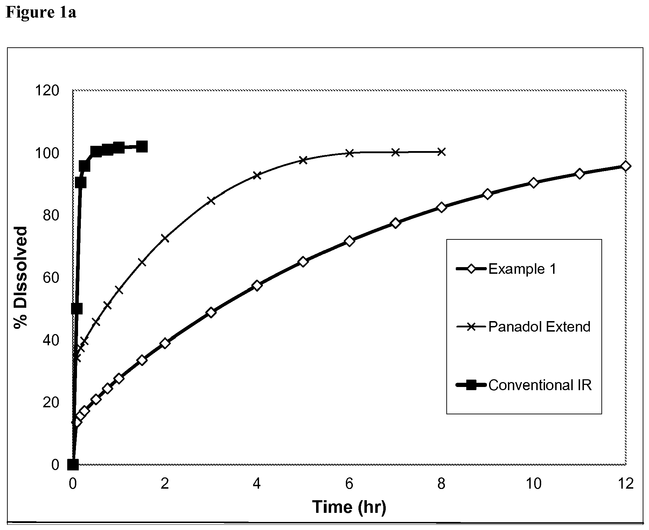

In one embodiment the present invention is an optionally twice daily administered unit dosage sustained release formulation of paracetamol that provides the following biorelevant dissolution profile, see Figure 1a.

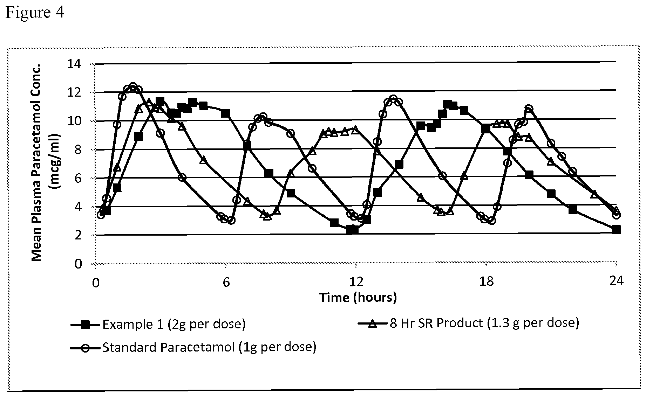

In one embodiment the present inventive sustained release formulation has been found to be bioequivalent in the fed and fasted state to a 4 times daily conventional immediate release formulation and to a 3 times daily 8-hour extended release formulation Panadol Extend®. See Figure 4.

It should be recognized herein that all doses of the sustained release formulation administered herein comprise a 2000mg unit dosage of paracetamol, as administered.

The sustained release formulations of paracetamol as exemplified herein are comprised of two bilayer tablets, each tablet containing 1000mg of paracetamol. Compliance with a twice daily oral dosing regimen is recognized to be better than with a four times daily dosing regimen of an immediate release paracetamol product. Two systematic reviews, as described below, support this assertion showing good evidence that the number of dosing time points significantly affects compliance.

Greenberg' R., Clinical Therapeutics, 6(5):592-9 in 1984, included studies that used pill counts, interviews and measurements of drug substances in body fluids to ascertain compliance. The results demonstrate quite clearly that compliance is related to the dosing schedule. Importantly, while the difference between compliance with once- or twice-daily regimens does not greatly differ, reducing the regimen from four- to twice-daily improves compliance by nearly 30%.

Since the Greenberg report, methods of measuring compliance have improved. A second review published by Claxton et al. Clin. Ther, 23(8):1296-310) in 2001, included studies that used electronic monitoring. Electronic monitors use microprocessors to record the precise time that a dose is removed from the monitoring unit. They can record both the number of events, especially whether doses are missed, and whether doses are taken at the correct time.

The results obtained in this review demonstrate clear agreement between the newer and older methods of measuring compliance and both give clear evidence that reducing the dosing schedule from four- to twice-daily increases the rate of compliance.

A potential disadvantage concerning a formulation containing more than the standard dose of paracetamol (500 mg) is accidental or intentional overdose. In such circumstances more paracetamol will be ingested from a sustained release formulation compared to a conventional immediate release formulation for any given number of unit doses such as tablets. The large ingestion of paracetamol could have serious consequences for an overdose patient, especially if a large amount of the dose is absorbed before rescue therapy could be initiated. It would therefore be preferable if the unit dose (such as a tablet or capsule) was designed to limit the amount of paracetamol released and therefore absorbed in the first few hours following dosing. An advantageous sustained release formulation should therefore demonstrate a lower C*ₘₐₓ* than a conventional immediate release formulation which would be indicative of a lower initial exposure.

One possible consequence, however, of formulating an orally administered paracetamol product designed to have a lower C*ₘₐₓ* and a slower rate of absorption is that the extent of absorption may also be decreased. The present invention is believed to have overcome the potential issue of decreased absorption.

A further advantage for a product designed to have a lower C*ₘₐₓ* and a slower rate of absorption where the extent of absorption is essentially complete (as demonstrable by an equivalent dose corrected AUC compared to immediate release tablets) is that it should have the advantage of maintaining therapeutic levels of paracetamol in plasma for extended periods following dosing and hence provide analgesia for longer than a conventional immediate release tablet or capsule. Furthermore as a result of the reduced C*ₘₐₓ* in the fasted state, the product of the present invention shows systemic levels of paracetamol remaining at more constant levels, thus benefiting the patient. The fluctuation index of the present invention has been shown to be lower than a conventional immediate release formulation.

While a formulation should have a lower C*ₘₐₓ* compared to conventional immediate release formulations, it is still desirable to also have a fast onset of action. The present invention demonstrated that initial levels of paracetamol in plasma were rapidly attained (preferably within 30 minutes) and were maintained at therapeutic levels for a period of time long enough for the release and absorption of paracetamol from the sustained release phase to start to take effect and maintain therapeutic levels for up to 12 hours.

As used herein a therapeutic level constitutes a level of paracetamol in the plasma of the patient of at least about 3ug/ml. In another embodiment of the invention a therapeutic level of paracetamol constitutes at least about 4ug/ml. In yet another embodiment of the invention a therapeutic level of paracetamol constitutes at least about 5ug/ml.

In one embodiment, the sustained release formulations provide a therapeutic plasma level of paracetamol of at least 3ug/ml for about 10 hours. In another embodiment of the invention the sustained release formulation provides a therapeutic plasma level of paracetamol of at least 4ug/ml for 8 about hours. In yet another embodiment, the sustained release formulation provides a therapeutic plasma level of paracetamol of at least 5ug/ml for about 6 hours.

In one embodiment, the sustained release formulation provides the time duration of plasma paracetamol concentration at or above therapeutic level (≥4µg/ml) for a single dose of 2000mg sustained release paracetamol which is about double that for a single dose of 1000mg immediate release paracetamol. Suitably, the time duration of plasma paracetamol concentration at or above therapeutic level (≥4µg/ml) for a single dose of 2000mg sustained release paracetamol is about 8.0-8.6 hrs and the time duration of plasma paracetamol concentration at or above therapeutic level (≥4µg/ml) for a single dose of an immediate release 1000mg dose of paracetamol is about 4.0-4.2 hrs.

In one embodiment, the sustained release formulation provides a time duration of plasma paracetamol concentration at or above therapeutic level (≥4µg/ml) for a single dose of 2000mg sustained release paracetamol which is 36 to 46% greater than that for a single dose of an extended release 1330mg dose of paracetamol, formulated for administration three times daily. Suitably, the time duration of plasma paracetamol concentration at or above therapeutic level (≥4µg/ml) for a single dose of 2000mg sustained release paracetamol is about 8.0-8.6 hrs and the time duration of plasma paracetamol concentration at or above therapeutic level (≥4µg/ml) for a single dose of an extended release 1330mg dose of paracetamol is about 5.9-6.2 hrs.

In one embodiment, the sustained release formulation provides a median time to maximum plasma concentration (T*ₘₐₓ*) of the paracetamol from about 3 hours to about 6.5 hours after administration of a single dose of 2000mg sustained release paracetamol.

In one embodiment, the sustained release formulation provides a width (time duration) at or above 50% of the height of a mean plasma concentration/time curve of the paracetamol from about 7 hrs to about 9 hrs after administration of a single dose of 2000mg sustained release paracetamol.

In one embodiment, the sustained release formulation provides a maximum mean plasma concentration (C*ₘₐₓ*) of the paracetamol which is more than about 3 to about 4 times the minimum mean plasma level concentration (C*ₘᵢₙ*) of paracetamol at about 12 hours after administration of a single dose of 2000mg sustained release paracetamol.

In one embodiment, the sustained release formulation provides a mean plasma concentration (C*ₘₐₓ*) of the paracetamol of from about 6.3µg/ml to about 17.1µg/ml, based on administration of a single dose of 2000mg sustained release paracetamol. Suitably, the mean plasma concentration (C*ₘₐₓ*) of the paracetamol is from about 8.9µg/ml to about 12.5µg/ml, based on administration of a single dose of 2000mg sustained release paracetamol. Suitably, the mean plasma concentration (C*ₘₐₓ*) of the paracetamol is from about 8µg/ml to about 13µg/ml, based on administration of a single dose of 2000mg sustained release paracetamol.

In one embodiment the sustained release formulation provides a mean plasma concentration (C*ₘₐₓ*) of the paracetamol of from about 9µg/ml to about 17µg /ml, based on administration of a repeat dose (steady state) of 2000mg sustained release paracetamol.

In one embodiment, there is a sustained release formulation of paracetamol comprising a sustained release phase of paracetamol and an immediate release phase of paracetamol, the formulation having single dose pharmacokinetic characteristics in fasted and fed states:
a) a plasma level of paracetamol which is at or above at least about 4µg/ml for a mean duration of about 8 hours; and
b) wherein the geometric mean AUC_{(0-∞)} is about 100 µg*h/ml to 104 µg*h/ml in the fasted state and about 99 µg*h/ml to 103 µg*h/ml in the fed state; and
c) the amount of paracetamol administered is 2000mg, as compared to a single 1000mg dose of immediate release paracetamol formulated for administration every 4-6 hours or as compared to a single 1330 mg dose of an 8-hour extended release paracetamol formulated for administration every 8 hours.

In yet another embodiment, the sustained release formulation according to the formulations described herein has single dose pharmacokinetic characteristics in the fasted and fed state of:
a) a mean AUC_{(0-∞)} is about 77 µg*h/ml to about 133 µg*h/ml (or more); and
b) a Kₑₗ is about 0.5 to about 0.13 hr-1 in fasted state or a Kₑₗ of about 0.09 to about 0.17 hr -1 in fed state; and
c) the amount of paracetamol administered is 2000mg, as compared to a single 1000mg dose of immediate release paracetamol formulated for administration every 4-6 hours or as compared to a single 1330 mg dose of an 8-hour extended release paracetamol formulated for administration every 8 hours.

Suitably, the mean AUC_{(0-∞)} is about 77 µg*h/ml to 133 µg*h/ml in the fasted state based upon administration of a 2000mg sustained release dose of paracetamol as defined above. Suitably, the mean AUC_{(0-∞)} is about 85 µg*h/ml to 120 µg*h/ml in the fasted state based upon administration of a 2000mg sustained release dose of paracetamol as defined above. Suitably, the mean AUC_{(0-∞)} is about 95 µg*h/ml to 115 µg*h/ml in the fasted state based upon administration of a 2000mg sustained release dose of paracetamol as defined above. Suitably, the geometric mean AUC_{(0-∞)} is about 100 µg*h/ml to 110 µg*h/ml in the fasted state.

In an alternative embodiment there is a sustained release formulation of paracetamol comprising a sustained release phase of paracetamol and an immediate release phase of paracetamol, the formulation having single dose pharmacokinetic characteristics in fasted and fed states:
a) a plasma level of paracetamol which is at or above at least 4µg/ml for about a mean duration of about 8 hours; and
b) wherein the Kₑₗ is about 0.09 hr⁻¹ in fasted state and the Kₑₗ is about 0.13 hr⁻¹ in fed state; and
c) the amount of paracetamol 2000mg is administered as compared to a single 1000mg dose of immediate release paracetamol, formulated for administration every 4-6 hours, or compared to a single 1330 mg dose of an extended release formulation of paracetamol, formulated for administration every 8 hours.

In another embodiment, there is a sustained release formulation of paracetamol comprising a sustained release phase of paracetamol and an immediate release phase of paracetamol, the formulation having single dose pharmacokinetic characteristics in fasted and fed states:
a) a plasma level of paracetamol which is at or above at least 4µg/ml for about a mean duration of about 8 hours; and
b) wherein the mean AUC_{(0-∞)} is about 100 µg*h/ml to 104 µg*h/ml in the fasted state and about 99 µg*h/ml to 103 µg*h/ml in the fed state; and
c) wherein the Kₑₗ is about 0.09 hr⁻¹ in fasted state and the Kₑₗ is about 0.13 hr⁻¹ in fed state; and
d) the amount of paracetamol administered is 2000mg, as compared to a single 1000mg dose of immediate release paracetamol formulated for administration every 4-6 hours or as compared to a single 1330 mg dose of an 8-hour extended release paracetamol formulated for administration every 8 hours.

In one embodiment, the mean AUC₍₀₋₂₄₎ of the sustained release formulation described herein at steady state is between about 124 µg*h/ml and about 204 and µg*h/ml; with a mean of about 165 µg*h/ml. In comparison, the mean AUC₍₀₋₂₄₎ of an immediate release formulation of paracetamol is between about 124 µg*h/ml and about 212 µg*h/ml; with a mean of about 168 µg*h/ml.

In one embodiment, the mean AUC₍₀₋₂₄₎ of the sustained release formulation described herein for a single dose is between about 64 µg*h/ml and about 124 µg*h/ml; with a mean of about 86-89 µg*h/ml in the fasted state. Suitably, the mean is about 95-97 µg*h/ml in the fed state.

In one embodiment, the mean AUC_{(0-∞)} of the sustained release formulation described herein at steady state is between about 133 µg*h/ml and about 217 µg*h/ml; with a mean of about 175 µg*h/ml. In comparison, the mean AUC_{(0-∞)} of an immediate release formulation of paracetamol is between about 129 µg*h/ml and about 225 µg*h/ml; with a mean of about 177 µg*h/ml.

In one embodiment, the mean AUC₍₀₋₆₎ of the sustained release formulation described herein in a fasted state is between about 29 g^{*}h/ml and about 51 µg*h/ml; with a mean of about 40 µg*h/ml. In comparison, the mean AUC₍₀₋₆₎ of an immediate release formulation of paracetamol is between about 31 µg*h/ml and about 51 µg*h/ml; with a mean of about 41 µg*h/ml.

In one embodiment, the mean AUC₍₀₋₆₎ of the sustained release formulation described herein in a fed state is between about 24 µg*h/ml and about 52 µg*h/ml; with a mean of about 38 µg*h/ml. In comparison, the mean AUC₍₀₋₆₎ of an immediate release formulation of paracetamol is between about 25 µg*h/ml and about 40 µg*h/ml; with a mean of about 33 µg*h/ml.

In another embodiment, a sustained release formulation having the above noted characteristics has an immediate release phase that produces or provides to the patient a therapeutic plasma concentration of paracetamol of 4µg/ml in about 0.5 hours (median time).

Upon multiple dosing of a formulation of the present invention, steady state plasma levels of paracetamol should be more constant than those achieved following multiple dosing of a conventional immediate release formulation. A convenient measure of the fluctuation in plasma concentrations is the fluctuation index (FI) which is defined as (C*ₘₐₓ*-C*ₘᵢₙ)*/C*_{average}.* A low FI number is considered to be advantageous as it suggests a reduction in the variability of plasma concentrations which is indicative of a safer product. A clinical study has demonstrated that a formulation of the present invention provides a slightly lower mean C*ₘₐₓ* and a smaller FI, showing less fluctuation in paracetamol plasma concentrations, as compared to a conventional immediate release formulation (FI mean value of 1.4 as compared to 1.5 for a conventional immediate release formulation). This study appears to indicate that the present formulation has less fluctuation in paracetamol plasma concentrations over a 24 hour period when dosed every 12 hours as compared to a conventional immediate release formulation dosed every 6 hours (2x 1000mg every 12 hours vs. 2 x 500 mg every 6 hours).

The formulation of the present invention showed a greater fluctuation than Panadol® Extend (FI mean value of 1.4 as compared to 1.2) in paracetamol plasma concentrations.

In one embodiment, the sustained release formulations described herein provide a mean AUC₍₀₋₂₄₎ or mean AUC_{(0-∞)} of at least 80% to about 125% of the mean AUC₍₀₋₂₄₎ or mean AUC_{(0-∞)} as provided by administration of 1000mg of an immediate release reference standard 4 times daily, wherein the daily dose of the reference standard is substantially equal to a twice daily dose of the sustained release paracetamol formulation.

Suitably, the sustained release formulation described herein provides a mean AUC₍₀₋₂₄₎ or mean AUC_{(0-∞)} of at least about 95% to about 105% of the mean AUC₍₀₋₂₄₎ or mean AUC_{(0-∞)} provided by administration of 1000mg of an immediate release reference standard 4 times daily, wherein the 4 times daily dose of the reference standard is substantially equal to a twice daily dose of 2000mg of the sustained release paracetamol formulation.

In another embodiment, the sustained release formulations as described herein provide a mean AUC₍₀₋₆₎ of at least 95% to about 105% of the mean AUC₍₀₋₆₎ provided by administration of 1000mg of an immediate release reference standard 4 times daily, wherein the daily dose of the reference standard is substantially equal to a twice daily dose of 2000mg of the sustained release paracetamol formulation.

Suitably, the sustained release formulations as described herein provide a mean AUC₍₀₋₆₎ of at least 85% to about 115% of the mean AUC₍₀₋₆₎ provided by administration of 1000mg of an immediate release reference standard 4 times daily, wherein the daily dose of the reference standard is substantially equal to a twice daily dose of 2000mg of the sustained release paracetamol formulation (in the fed state).

In a clinical study, a single 2000mg dose of a formulation of the present invention demonstrated greater than 90% relative bioavailability as compared to a single 1000mg dose of a conventional immediate release paracetamol formulation based upon a dose corrected adjustment.

In a second clinical study, a single 2000mg dose of the formulation of the present invention demonstrated greater than 90% relative bioavailability as compared a single 1330mg dose of an 8-hour extended release formulation, Panadol Extend®, based upon a dose corrected adjustment.

It has now been found that these advantageous pharmacokinetic profiles can be provided by a two phase (immediate release and sustained release) formulation of paracetamol that satisfies not only a unique *in vitro* dissolution profile, but also has a unique *in vivo* pharmacokinetic profile.

In an alternative embodiment there is a sustained release formulation of paracetamol comprising a sustained release phase of paracetamol and an immediate release phase of paracetamol, the formulation having repeat dose pharmacokinetic characteristics:
a) a plasma level of paracetamol which is at or above at least about 4µg/ml for about a mean duration of about 16 hours, suitably 17 hours (during 24 hours at steady state);
b) wherein the mean AUC_{(0-∞)} is about 173 µg*h/ml at steady state (when administered twice daily);
c) wherein the 90% confidence intervals for the ratios of the formulation versus an 8-hour extended release formulation, and the formulation versus a conventional immediate release formulation, for three pharmacokinetic parameters (AUC0-t, AUC0-inf, and Cmax) all lie within the bioequivalence boundaries (0.8, 1.25);
d) the amount of paracetamol administered is 2000mg twice a day for three days, as compared to 1000mg of immediate release paracetamol four times a day for three days or as compared to 1330 mg of 8 hours paracetamol three times a day for three days.

Suitably, a sustained release formulation as described herein provides a mean AUC_{(0-∞)} in a patient in a range between about 173 µg*h/ml to 175 µg*h/ml at steady state (when the unit dosage of 2000mg is administered twice daily).

In an alternative embodiment there is a sustained release formulation of paracetamol comprising a sustained release phase of paracetamol and an immediate release phase of paracetamol, the formulation having repeat dose pharmacokinetic characteristics:
a) a plasma level of paracetamol which is at or above at least about 4µg/ml for a mean duration of about 16 hours, suitably 17 hours (during 24 hours at steady state);
b) wherein the Kₑₗ is about 0.26 hr⁻¹; and
c) wherein the fluctuation index FI is about 1.4; and
d) the amount of paracetamol administered is 2000mg twice a day for three days, as compared to 1000mg of immediate release paracetamol four times a day for three days or as compared to a 1330 mg dose of an 8-hour extended release paracetamol formulation three times a day for three days.

In an alternative embodiment there is a sustained release formulation of paracetamol comprising a sustained release phase of paracetamol and an immediate release phase of paracetamol, the formulation having repeat dose pharmacokinetic characteristics:
a) a plasma level of paracetamol which is at or above at least 4µg/ml for about a mean duration of 16 hours, suitably about 17 hours (during 24 hours at steady state);
b) wherein the mean AUC_{(0-∞)} is about 173µg*h/ml at steady state (when administered twice daily);
c) wherein the 90% confidence intervals for the ratios of the formulation versus an 8-hour extended release formulation, and the formulation versus a conventional immediate release formulation, for three pharmacokinetic parameters (AUC0-t, AUC0-inf, and Cmax) all lie within the bioequivalence boundaries (0.8, 1.25);
d) wherein the Kₑₗ is about 0.26 hr⁻¹; and
e) wherein the fluctuation index FI is about 1.4; and
f) the amount of paracetamol administered is 2000mg twice a day for three days, as compared to 1000mg of immediate release paracetamol four times a day for three days or as compared to 1330 mg of 8-hours paracetamol three times a day for three days.

Suitably, the formulation described herein provides a mean AUC_{(0-∞)} in a patient in a range between about 173 µg*h/ml and 175 µg*h/ml at steady state (when administered twice daily). The present invention is directed to a sustained release formulation for oral administration containing 2000mg of paracetamol per unit dose administered as two bilayer tablets, each tablet containing 1000mg of paracetamol present in a sustained release phase and in an immediate release phase, in which the ratio of the paracetamol in the sustained release phase to the immediate release phase is 80-90% to 10-20%, and wherein the sustained release phase comprises 4% to 6% by weight of at least one high viscosity hypromellose, 0.5% to 3% by weight of at least one binding agent which is povidone, from 1% to 2% by weight of a low viscosity hypromellose, 0.5% to 3% by weight of at least one modified starch, and wherein the ratio of high-viscosity hypromellose to low viscosity hypromellose is 3.3 to 0.85, wherein when ingested by a human, the formulation reduces the maximum attained plasma-paracetamol concentration by at least about 4.5% at steady state (relative to rapid-release paracetamol formulations'), and increases the time to reach a maximum paracetamol-plasma concentration (T*ₘₐₓ*) by at least about 140% at steady state (relative to rapid-release paracetamol formulations'), while having an insignificant effect on area under the plasma-paracetamol concentration time curve AUC₍₀-₂₄₎; mean AUC₍₀₋₂₄₎ of about 165 µg*h/ml for sustained release paracetamol at steady state (2000mg dosed every 12 hours) versus a mean AUC₍₀₋₂₄₎ of about 168 µg*h/ml for 1000mg immediate release at steady state (dosed every 6 hours) and wherein the formulation is repeatedly administered (steady state). The above sustained release formulation for oral administration provides a therapeutically effective plasma paracetamol concentration over a 12 hour period for use in the treatment of pain in a human in need thereof wherein the formulation has the following in vitro bio-dissolution profile of the dissolution release ranges at various time points (as determined by USP Type II apparatus, rotating paddle, with 900 ml of Phosphate buffer at pH 7.4, 37 °C set at rotating speed of 75rpm) of:
2 to 15% released at 15 minutes;
4 to 22% released at 30 minutes;
10 to 40% released at 60 minutes;
22 to 62% released at 180 minutes;
50 to 88% released at 360 minutes;
>90% released after 720 minutes.

In an alternative embodiment 15 to 50% is released at 120 minutes.

In an alternative embodiment 28 to 70% is released at 240 minutes.

In an alternative embodiment 81 to 100% is released at 600 minutes.

In another embodiment there is an *in vitro* bio-dissolution profile of the sustained release formulations described herein, having these pharmacokinetic parameters, which will also have the following dissolution release range at various time points (as determined by USP Type II apparatus, rotating paddle, with 900 ml of Phosphate buffer at pH 7.4, 37 °C set at rotating speed of 75rpm) of:
- 2 to 15% released at 15 minutes;
- 4 to 22% released at 30 minutes;
- 10 to 40% released at 60 minutes;
- 15 to 50% released at 120 minutes;
- 22 to 62% released at 180 minutes;
- 28 to 70% released at 240 minutes;
- 50 to 88% released at 360 minutes;
- 81 to 100% released at 600 minutes; and
- >90% released after 720 minutes.

Many physiological factors influence both the gastrointestinal transit time and the release of a drug from a controlled release dosage form, and thus influence the uptake of the drug into the systemic circulation. A sustained-release dosage form should release the paracetamol at a controlled rate such that the amount of active ingredient available in the body to treat the condition is maintained at a relatively constant level over an extended period of time. That is, it is desirable that paracetamol be released at a reproducible, predictable rate which is substantially independent of physiological factors which can vary considerably among different individuals and even over time for a particular individual.

The release of an active ingredient from a controlled release dosage form is generally controlled either by diffusion through a coating, diffusion of the agent from a monolithic device, or by erosion of a coating by a process which is dependent upon enzymes or pH. Because such factors can vary from time to time for a particular individual, and can also vary from one individual to another, enzymes or pH dependent sustained-release pharmaceutical formulations generally may not provide a reproducible rate of release of the active pharmaceutical ingredient. Thus these types of formulations do not minimize intra-subject and inter-subject variation in bioavailability of the active ingredient.

As can be shown by many failed experiments, previous formulations fail to maintain a median therapeutic plasma level (e.g., 3 - 5µg/ml) in the body for sufficient time (i.e., 10 or more hours). In some formulations, the resulting tablet sizes containing 1000mg APAP were very large and could be highly inconvenient for the patient to swallow. The ability to maintain a therapeutic level of paracetamol (e.g., 3 - 4µg/ml) in the body over a period of at least 10 hours in a 2 tablets per dose formulation and in a tablet size that is readily swallowable has been virtually impossible until the present invention.

Similarity factor (f2) is a recognized method for the determination of the similarity between the dissolution profiles of a reference and a test compound. Similarity factor (f2) is a logarithmic transformation of the sum of squared error. The similarity factor (f2) is 100 when the test and reference profiles are identical and approaches zero as the dissimilarity increases. The similarity factor has also been adapted to apply to the determination of the similarity between the dissolution profiles of a reference and test compound as they relate to modified release formulations, such as those exemplified herein.

The f2 similarity factor has been adopted in the SUPAC guidelines and by the FDA guidance on dissolution testing of immediate release dosage forms (FDA Guidance for Industry, Dissolution Testing of Immediate Release Solid Oral Dosage Forms, FDA, (CDER), August 1997 (Dissolution Tech. 4, 15-22, 1997).

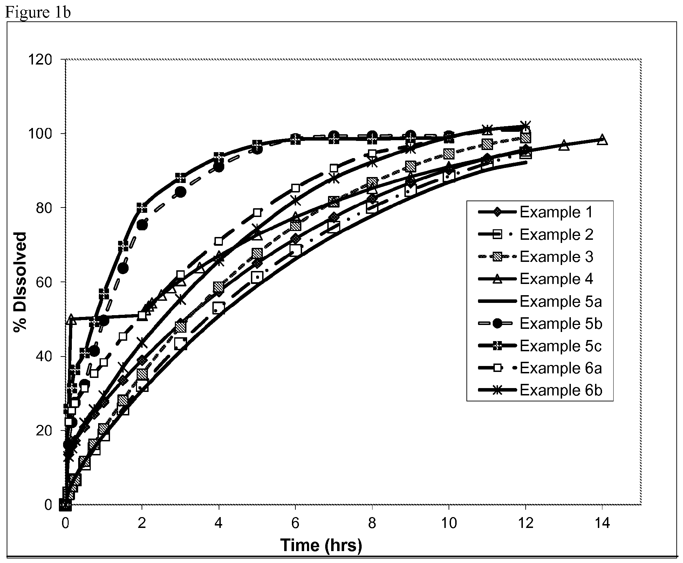

The f2 similarity factor has been adopted in the FDA in the SUPAC guidelines for modified release solid oral dosage forms (FDA Guidance for Industry, SUPAC-MR: Modified Release Solid Oral Dosage Forms, Scale-Up and Postapproval Changes: Chemistry, Manufacturing, and Controls; In Vitro Dissolution Testing and In Vivo Bioequivalence Documentation; CDER; September 1997). The FDA Guidance for Industry on Dissolution Testing of Immediate Release Solid Oral Dosage Forms may be found at http://www.fda.gov/cder/guidance/1713bp1.pdf. The sustained release dosage form of the present invention comprises a bi-layer tablet having a sustained release (SR) phase layer and an immediate release phase layer. The SR phase contains a therapeutically effective amount of paracetamol, suitably in granulate form. The immediate release phase and the sustained release phase will both contain paracetamol and other pharmaceutically acceptable carriers and functional excipients that are suitably combined together into the separate layers of a bi-layer tablet. The present invention is also directed to formulations of sustained release paracetamol that have an *in vitro* dissolution profile generated using the USP Type II apparatus, rotating paddle method as described herein with a similarity factor (f2) between 50 and 100 when calculated using one of the examples of the present invention described herein in Figure 1a and 1b as the reference profile. Examples 2 to 5c in Fig. 1b are outside the scope of the invention. Herein it is also disclosed, but does not form part of the invention, a sustained release dosage form which is a single layer unit (a monolith tablet) having a sustained release phase of paracetamol and an immediate release phase of paracetamol. Suitably, in one embodiment the sustained release phase is a separate blend, granules or pellets which form an intragranular component, and the immediate phase can be comprised of separate blends, granules or pellets to form the extragranular component. The immediate release phase and the sustained release phase can be then admixed together with any other pharmaceutically acceptable excipients desired before being compressed into a single layer tablet. Herein it is also disclosed, but does not form part of the invention, a sustained release dosage form which is a single layer unit having a therapeutically effective amount of paracetamol present only as a sustained release intragranular phase. The extragranular phase comprises a non-water soluble matrix forming polymer and other suitable carriers and excipients. This dosage form does not have an immediate release component of paracetamol.

As used herein granulate is a material that has been adapted and preprocessed by suitable means such as slugging, aqueous or non-aqueous wet granulation, fluidized bed granulation, spray drying or roller compaction to form granules. For purposes herein, a component of the granulate is referred to as "intragranular" or an "intragranular component", whereas a component that is admixed with said granulate is referred to as "extragranular" or an "extragranular component".

Suitably, the paracetamol in either the bilayer or monolith dosage form is approximately a 80:20 ratio of sustained release to immediate release amounts of paracetamol. In another embodiment, the paracetamol is in an approximate 90:10 ratio of sustained to immediate release amounts. Explained differently, for a 1000mg containing tablet, the paracetamol is present in an amount of about 900mg in the sustained release phase and about 100mg in the immediate release phase. For example, in one embodiment, the ratios of sustained release to immediate release phase represent the proportional amount of each layer in a bi-layer dosage form. In another embodiment, the ratios represent the amount of paracetamol in the sustained release intragranular component versus the immediate release extragranular component of a single layer dosage form, which is not according to the invention. In another embodiment (outside the scope of the invention), the amount of sustained release intragranular component containing paracetamol is 100%. In this embodiment there is no immediate release phase. See a representative example of this in Table 3, Example 3, which is not according to the invention. The sustained release layer of the bilayer tablets comprises at least one high viscosity hypromellose (HPMC) ingredient. HPMC is a water soluble matrix-forming polymer used to provide a sustained release effect of paracetamol. Suitably the viscosity of the HPMC used in the SR phase is in the range of about 3500-6000 centipoise.

It will be understood by the skilled artisan that the high viscosity HPMC can suitably be a blend of multiple high viscosity HPMC's resulting in a total overall range of 3500-6000 centipoise.

The amount of matrix-forming polymer in the sustained release phase and the relative amounts of paracetamol in the sustained release and immediate release phases are selected so as to provide the desired *in vitro* dissolution rate as described herein.

In accordance with the invention, there is a bilayer tablet having a sustained release layer and an immediate release layer. The sustained release layer comprises a therapeutically effective amount of paracetamol, 4% to 6% by weight of at least one high viscosity hypromellose, 0.5% to 3% by weight of at least one binding agent which is povidone, from 1% to 2% by weight of a low viscosity hypromellose, 0.5% to 3% by weight of at least one modified starch, and optionally one or more other pharmaceutically acceptable intragranular components including but not limited to a second pharmaceutically acceptable active ingredient, other pharmaceutically acceptable excipients and/or adjuvants. The ratio of high-viscosity hypromellose to low viscosity hypromellose is 3.3 to 0.85. In another embodiment the ratio of high to low is about 3:1. For representative examples of this range, see Working Example 1. Suitably, the viscosity of the low viscosity hypromellose is in the range of about 10-30 centipoises. In another embodiment the low viscosity is about 15 centipoises. The amount of at least one binding agent in the sustained release phase of the bilayer tablet is from 0.5% to 3% w/w. In one embodiment there are at least two binding agents present in the SR phase. The amount of at least one modified starch in the sustained release phase of the bilayer tablet is from 0.5% to 3% w/w. In one embodiment, the amount of modified starch is about 1% w/w of the SR phase. In one embodiment there are at least two modified starches present in the SR phase. Suitably, the modified starch is pre-gelatinized. The amount of the high viscosity hypromellose present in the sustained release phase is from 4% to 6% of the sustained release phase formulation weight. In yet another embodiment the amount of high viscosity HPMC is present in an amount of about 5% w/w of the sustained release phase formulation weight. The amount of the low viscosity hypromellose present in the sustained release phase is from 1% to 2% of the sustained release phase formulation weight. In another embodiment the amount of low viscosity HPMC is of about 1.6% w/w of the sustained release phase formulation weight.

In one embodiment the SR phase comprises paracetamol, povidone, pre-gelatinized corn starch, and a high and low viscosity HPMC.

The immediate release layer may be prepared by combining a directly compressible commercially available grade of paracetamol with a lubricant, and one or more disintegrating agents if necessary or desired. Binders and other excipients and/or adjuvants may be included in the immediate release layer, also if necessary or desired. Paracetamol in the immediate release layer is generally combined with a modified starch such as a pre-gelatinized starch, e.g., corn starch, a disintegrant, or super disintegrant such as croscarmellose sodium or Explotab®, a binder and a lubricant.

### Monolith Dosage Form

Also herein disclosed (not according to the invention) is only a single layer tablet having a sustained release intragranular phase and an immediate release extragranular phase. The sustained release phase will be comprised of an intragranular component of paracetamol and a high viscosity hypromellose as defined above, at least one binding agent, a low viscosity hypromellose as defined above, at least one modified starch, and optionally one or more other pharmaceutically acceptable intragranular components including but not limited to a second pharmaceutically acceptable active ingredient, and optional excipients and/or adjuvants. These components form the SR granulate. The SR blend could be made into pellets and compressed accordingly with the extragranular immediate release blend.

A suitable amount of the high viscosity hypromellose present in the sustained release phase granulate is from about 3% to about 7% of the sustained release phase formulation weight. In another embodiment, the amount of high viscosity hypromellose is from about 4% to about 6% of the sustained release phase formulation weight. In another embodiment, the amount of high viscosity HPMC is present in an amount of about 5% w/w of the sustained release phase formulation weight.

Suitably, the amount of the low viscosity hypromellose present in the sustained release phase granulate is from about 0.5% to about 3% of the sustained release phase formulation weight. In another embodiment, the amount of low viscosity hypromellose is from about 1% to about 2% of the sustained release phase formulation weight. In another embodiment the amount of low viscosity HPMC is of about 1.6% w/w of the sustained release phase formulation weight.

Alternatively, the total amount of cellulosic derivatives of HPMC present in the SR granulate range from about 3% to about 10% by weight of the total amount of sustained release components. This encompasses both the high and the low viscosity HPMC's.

Suitably the amount of at least one binding agent in the sustained release phase granulate is from about 0.5% to about 3% w/w. In one embodiment there are at least two binding agents present in the SR granulate.

Suitably the amount of at least one modified starch in the sustained release phase granulate is from about 0.5% to about 3% w/w. In one embodiment there are at least two modified starches present in the SR granulate.

A suitable extragranular component or phase, i.e., the immediate release phase, may be prepared by combining a directly compressible commercially available grade of paracetamol with a lubricant, and one or more disintegrating agents if necessary or desired. Binders and other excipients and/or adjuvants may be included in the extragranular phase if necessary or desired. Alternatively, an extragranular component can be prepared by combining paracetamol with a modified starch, such as a pre-gelatinized starch, e.g., corn starch, a disintegrant or super disintegrant, such as croscarmellose sodium, a binder and a lubricant. Most commercially available blends of immediate release paracetamol will be satisfactory for this component.

### Alternative Monolith Dosage Form

In monolith dosage forms not according to the invention, the ratio of high-viscosity hypromellose to low viscosity hypromellose can be altered to be about a 2:1 ratio. A suitable amount of the high viscosity hypromellose present in the sustained release phase is from about 0.5% to about 4% of the sustained release phase formulation weight. A suitable amount of low viscosity hypromellose in the sustained release phase is from about 0.5% to about 3% by weight of the sustained release phase. When the ratio of the HPMC's are altered, the extragranular phase will necessarily be comprised of a second, and different sustained release polymer, such as Kollidon® SR (BASF) suitably, in the amount of 4-8% by weight of the total composition. Kollidon® SR is derived from a polyvinyl acetate-dispersion (such as Kollicoat SR 30D) and is a powder consisting of polyvinyl acetate (8 parts w/w) and polyvinyl pyrrolidone (2 parts w/w).

The extragranular phase also includes a suitable lubricant, and optionally a second pharmaceutically acceptable active ingredient, and any other optional excipients and/or adjuvants as needed or desired. A representative example of this type of formulation is shown in Example 3 herein. This example is not according to the invention.

### Excipients

The present invention includes the binding agent povidone.

Suitably, the starch is of vegetable origin, such as modified corn starch, modified wheat starch, potato starch, or pre-gelatinized starch e.g., available commercially as Starch 1500 G or Prejel; or a combination of two or more thereof.

The term "low to medium" viscosity as used herein means a viscosity in the range of from about 15 to about 1000 mPas. It is recognized in the art that the determination of the viscosity of cellulosic derivatives is based upon standard techniques and grading in the art e.g., for HPMC, viscosity may be determined at 20°C with a 2% solution using a Ubbelohde viscometer, or for HPC, viscosity may be determined at 25°C with a 2-10% solution using a Brookfield LVF viscometer. It is recognized in the art that certain cellulosic derivatives, such as hypromellose, will have varying roles in a formulation, depending upon the amount used. For example hypromellose (low or medium viscosity) may function as a binding agent, a coating agent, or as a matrix forming agent.

While a binding agent is present as an intragranular component, it is recognized that a modest amount of binding agent e.g., up to about an additional 3.0%- 10.0% by weight of the intragranular binding agent content of the composition, may also be present extragranularly.

It is recognized that the present invention also requires a modified starch to be present. As modified starches can also be binding agents there will be at least two different components present in the particular phase.

In one embodiment, suitably the starch is pre-gelatinized starch. Pre-gelatinized starch is a starch that has been chemically and/or mechanically processed. Typically pre-gelatinized starch contains 5% of free amylase, 15% of free amylopectin, and 80% unmodified starch. Pre-gelatinized starch may be obtained from corn (or maize), potato or rice starch.

The granulate provides an intimate admixture of a combination of ingredients and may then be mixed with one or more pharmaceutically acceptable extragranular components of the composition i.e., with any pharmaceutically acceptable ingredient e.g., a diluent, flavor, sweetening agent, binder, disintegrant, glidant, lubricant, anti-adherent, anti-static agent, anti-oxidant, desiccant, or a second pharmaceutically acceptable active agent. It is recognized that these same ingredients may be present both as an intragranular and as an extragranular ingredient.

As noted above there are other inactive ingredients that may optionally be employed in relatively small quantities, and which do not affect the fundamental and essential characteristics of the invention which include lubricants, flow agents, and binders that facilitate compression.

Suitable disintegrating agents include a non-super disintegrant, a super disintegrant or a combination of both. Suitable non-super disintegrants include conventional disintegrants such as starch (corn or maize), pre-gelatinized starch e.g., Starch 1500 G, clays (Veegum or Bentonite), microcrystalline cellulose, cellulose or powdered cellulose. It is recognized in the art, that some excipients may perform more than one role in a given pharmaceutical formulation. For example certain excipients, e.g., starches including pre-gelatinized starch, and microcrystalline cellulose (hereinbefore identified as binding agents) function as both binders and disintegrants.

A "super disintegrant" represents a class of disintegrating agent which may generally be used in lower amounts in pharmaceutical preparations, as compared to conventional disintegrants. Examples of super disintegrants include sodium starch glycolate, the sodium salt of carboxymethyl starch, modified cellulose and cross-linked polyvinyl pyrrolidone. Sodium starch glycolate is available commercially under the trade names Explotab® (Edward Mendell Co.), Primojel® (Generichem Corp) and Tablo® (Blanver, Brazil). An example of modified cellulose includes croscarmellose, the sodium salt of carboxymethyl cellulose. Croscarmellose is available commercially under the trade names AcDiSol® (FMC Corp.), Nymcel ZSX® (Nyma, Netherlands), Primellose® (Avebe, Netherlands), Solutab® (Blanver, Brazil). An example of a cross-linked polyvinyl pyrrolidone includes crospovidone, and is commercially available under the trade names Kollidon CL® or Kollidon CL-M (Basf Corp.), and Polyplasdone XL® (ISP Corp). A suitable super disintegrant includes croscarmellose sodium or sodium starch glycolate (Explotab) or a combination thereof. A super disintegrant may be used extragranularly, in an amount ranging from about 0.5% to about 5.0% by weight of the composition.

Suitable preservative or antimicrobial agents for use include potassium sorbate or a paraben, i.e., one or more hydroxy benzoic acid esters e.g., methyl, ethyl, propyl or butyl, suitably singularly or as mixtures. Parabens are commercially available under the Nipa® brand name, e.g., Nipasept® sodium.

Suitable lubricants include magnesium, calcium or sodium stearate, stearic acid or talc that may be added in suitable amounts. In one embodiment the lubricant is magnesium stearate.

Suitable flow agents include silicon dioxide (Cab-O-Sil, Syloid™), that may be added in an amount from about 0.5% to about 1% by weight.

The compressed tablet may further comprise a film coat e.g., hypromellose. Suitably the film coat is a transparent film coat e.g., a dye, although an opaque film coat e.g., as obtained when using a film coat in combination with an opacifier or a pigment such as titanium dioxide or a lake may also be used. For example one commercially available film coat is an Opadry coating system from Colorcon. In the bilayer tablet, the immediate release layer may be compressed directly on a previously compressed sustained release layer, or alternatively, the sustained release layer may be compressed onto a previously compressed immediate release layer.

In addition to paracetamol, compositions of the invention may also contain other pharmaceutically active agents. The term "pharmaceutically active agent" includes, but is not limited to, drugs, nutritional agents, as described herein. This term includes bioactive agents, active agents, therapeutic agents, or drug(s) as defined herein, and follows the guidelines from the European Union Guide to Good Manufacturing Practice. Such substances are intended to furnish pharmacological activity or other direct effect in the cure, mitigation, treatment, or prevention of disease or to affect the structure and function of the body. The pharmacological activity may be prophylactic, or for treatment of a disease state.

Drug substances include those intended for oral administration. A description of these classes of drugs and a listing of species within each class can be found in Martindale, The Extra Pharmacopoeia, Twenty-ninth Edition, The Pharmaceutical Press, London, 1989. The drug substances are commercially available and/or can be prepared by techniques known in the art.

Suitable pharmaceutically active agents include but are not limited to other analgesics such as codeine, hydrocodone, oxycodone, tramadol and propoxyphene; anti-inflammatory analgesics such as NSAIDs e.g., aspirin and ibuprofen; decongestants such as pseudoephedrine and phenylephrine; antitussives such as pholcodine and dextromethorphan; expectorants such as guaifenesin and bromhexine; diuretics such as pamabrom; non-sedating and sedating antihistamines such as diphenydramine, doxylamine and mepyramine; gastrointestinal agents such as metoclopramide; triptans such as sumatriptan; muscle relaxants such as methocarbamol. Compositions may also contain a pharmaceutically acceptable adjuvant, for example caffeine. Pharmaceutically active agents and adjuvants e.g., may be present intragranularly, extragranularly or both intragranularly and extragranularly.

The compositions of the present invention can be formulated by conventional methods of admixture such as granulating, blending, filling and compressing. For example, tablets can be produced by a wet granulation process, where the immediate release phase and sustained release phase are separately prepared. Suitably, for either the immediate release or sustained release phase, the active drug substance and excipients are screened and mixed in a high shear mixer granulator or fluid bed dryer. The blend is granulated by the addition of a granulating solution (typically purified water, disintegration agent dissolved/dispersed in purified water, or drug dissolved/dispersed in purified water or a suitable solvent) sprayed into the high shear mixer granulator or fluid bed dryer. If desired wetting agents e.g., surfactants can be added. The resulting granules (optionally pelletized) are dried usually with residual moisture of 1-5% by tray, fluid bed or microwave drying techniques. The dried granules are milled to produce a uniform particle size, the granules are blended with extragranular excipients as necessary, typically a lubricant and glidant (e.g., magnesium stearate, silicon dioxide). The separately prepared immediate release and sustained release granules are then compressed together using a rotary bilayer tablet press if desired.

If the dosage form is a single layer tablet, then the sustained release granules are admixed with the immediate release extragranular components and compressed together using a rotary tablet press, etc. These resulting tablets can all be coated in a pan coater typically with a 1-5% aqueous film coat, followed by a wax polishing. Single layer tablets are not according to the invention. Alternatively tablets can be produced by a direct compression process. Suitably the active drug substance and excipients for the immediate release and sustained release phases are separately screened and mixed in a suitable blender e.g., a cone, cube or V-blender. Other excipients are added as necessary, and further blended. The separately prepared immediate release and sustained release phases can be combined and compressed together using a rotary tablet press as hereinbefore described. The resulting tablets can be coated in a pan coater.

Tablets can also be prepared by using both methods of wet granulation and direct compression. For example the sustained release phase can be prepared by wet granulation as described herein, while the immediate release phase can be prepared by blending the excipients for direct compression. Furthermore commercially available blends of immediate release paracetamol are also available for direct compression such as the DC90 paracetamol supplied by Rhodia. The two phases can then be combined and compressed together as hereinbefore described.

A suitable dissolution rate profile for the product described herein is wherein at least 10-30% of the paracetamol is released from the composition at 30 minutes and wherein at least 80% of the paracetamol is released from the composition at 600 minutes as determined by a dissolution method that utilizes a USP paddle apparatus rotating at 75 rpm, employing 900ml of phosphate buffer at pH7.4.

Accordingly, a pharmaceutical composition, not according to the invention, having an immediate release phase and a sustained release phase of paracetamol in a bilayer tablet, said composition comprising about 1000mg of paracetamol per unit dose and a pharmaceutically acceptable carrier, is characterized in having an *in vitro* paracetamol dissolution profile (as determined by the USP type II apparatus, rotating paddle, with 900ml of Phosphate buffer at pH7.4, 37C set at rotating speed of 75 rpm) with the following constraints:
- 10 to 30% released after 30 minutes
- 20 to 40% released after 90 minutes
- 35 to 55% released after 180 minutes
- >80% released after 600 minutes.

In another embodiment, a pharmaceutical composition, not according to the invention, having an immediate release phase and a sustained release phase of paracetamol in a monolith tablet, said composition comprising about 1000mg of paracetamol per unit dose and a pharmaceutically acceptable carrier, is characterized in having an *in vitro* paracetamol dissolution profile (as determined by the USP type II apparatus, rotating paddle, with 900ml of Phosphate buffer at pH7.4, 37C set at rotating speed of 75 rpm) with the following constraints:
- 5 to 25% released after 30 minutes
- 15 to 35% released after 90 minutes
- 35 to 55% released after 180 minutes
- >80% released after 600 minutes.

In yet another embodiment, the *in vitro* dissolution profile has the following constraints:
- 5 to 25% released after 30 minutes
- 15 to 35% released after 90 minutes
- 30 to 50% released after 180 minutes
- >80% released after 600 minutes.

In yet a further embodiment, the *in vitro* dissolution profile has the following constraints:
- 10 to 30% released after 30 minutes;
- 20 to 40% released after 90 minutes;
- 35 to 55% released after 180 minutes; and
- >80% released after 600 minutes.

It was expected that an insufficient amount of paracetamol would be released from the immediate release layers of two tablets to rapidly reach 4µg/ml paracetamol in the plasma. Surprisingly, it has been discovered that this was not the case. A clinical study demonstrated that the median time to reach 4µg/ml in the plasma for the formulation of Example 1 was similar to 1000mg standard paracetamol in the fasted state.

The following non-limiting Examples illustrate the advantageous properties of the compositions of the present invention.

### EXAMPLE 1

In a first example, a bilayer extended release paracetamol tablet is prepared using the following ingredients:

### EXAMPLE - 1 (Bi-Layer Tablet)

| **No.** | **INGREDIENT** | **mg per tablet** |
|---|---|---|
| **A** | **Sustained Release Layer (SR Layer)** | |
| | Intragranular components: | |
| 1 | Paracetamol fine | 900.0 |
| 2 | Hypromellose (HPMC) 2208 4000 cP | 50.0 |
| 3 | Povidone K25 | 20.0 |
| 4 | Pregel starch fine | 10.0 |
| 5 | Hypromellose (HPMC) 2910 15 cP | 16.0 |
| | Extragranular components: | |
| 6 | Magnesium Stearate | 3.0 |

| **B** | **Immediate Release Layer (IR Layer)** | |
|---|---|---|
| 7 | Paracetamol (DC90) | 111.2 |
| | | |

| **Bi-Layer Tablet** | | |
|---|---|---|
| **A** | Sustained Release Layer (SR Layer) | 999.0 |
| **B** | Immediate Release Layer (IR Layer) | 111.2 |
| | **Final Tablet weight (mg)** | 1110.2 |

The total tablet weight is about 1100mg, with about 1000mg of paracetamol per tablet. Layer 1 has a total weight of about 1000mg (approx 900.0mg paracetamol), and Layer 2 has a total weight of about 110mg (about 100 mg paracetamol). The mixtures for each of the layers are prepared and the layers are compressed on a suitable type rotary bi-layer tablet press.

Manufacturing Instructions for Sustained Release Layer:
1. Mix Paracetamol, HPMC 2208, PVP, HPMC 2910 and Pregel Starch in a high shear granulator.
2. Add purified water while mixing.
3. Continue mixing until suitable granulation end point is attained.
4. Dry the granulation to the target % LOD.
5. Milled dry granulation using Co-mill equipped with suitable size screen
6. Add lubricant, magnesium stearate, and mix.
7. Blend is now ready for compression.

Manufacturing Instructions for Immediate release Layer:
1. DC 90 paracetamol is used as an immediate release layer.

### EXAMPLE 2 (not according to the invention)

In another example, a single layer extended release paracetamol tablet is prepared using the following ingredients:

### EXAMPLE - 2 (Single Layer Tablet)

| **No.** | **INGREDIENT** | **mg per tablet** |
|---|---|---|
| | **Intragranular Components:** | |
| 1 | Paracetamol fine | 900.0 |
| 2 | Hypromellose (HPMC) 2208 4000 cP | 50.0 |
| 3 | Povidone K25 | 20.0 |
| 4 | Pregel starch fine | 10.0 |
| 5 | Hypromellose (HPMC) 2910 15 cP | 16.0 |

| | **Extragranular Components:** | |
|---|---|---|
| 6 | Paracetamol (DC90) | 111.2 |
| 7 | Magnesium Stearate | 3.0 |
| | **Final Tablet weight (mg)** | 1110.2 |

The total tablet weight is about 1100mg, with about 1000mg of paracetamol per tablet. Manufacturing Instructions for Intragranular components:
1. Mix Paracetamol fine, HPMC 2208, povidone, HPMC 2910 and Pregel Starch in a high shear granulator.
2. Add purified water while mixing.
3. Continue mixing until suitable granulation end point is attained.
4. Dry the granulation to the target % LOD.
5. Milled dry granulation using Co-mill equipped with suitable size screen

Manufacturing instructions for final mix:
1. Add intragranular blend and paracetamol DC 90 to a suitable low shear blender and mix.
2. Add the lubricant, magnesium stearate, to the blender and mix.

The final mix is now ready for compression. Compress the tablets on a suitable size rotary tablet press.

### EXAMPLE 3 (not according to the invention)

In another example, a single layer extended release paracetamol tablet is prepared using the following ingredients:

### EXAMPLE - 3 (Single Layer Tablet with 6% Kollidon SR)

| **No.** | **INGREDIENT** | **mg per tablet** |
|---|---|---|
| | Intragranular Components: | |
| 1 | Paracetamol fine | 1000.0 |
| 2 | Hypromellose (HPMC) 2208 4000 cP | 32.6 |
| 3 | Povidone K25 | 21.7 |
| 4 | Pregel starch fine | 10.9 |
| 5 | Hypromellose (HPMC) 2910 15 cP | 17.4 |

| | Extragranular Components: | |
|---|---|---|
| 6 | Kollidon SR | 69.3 |
| 7 | Magnesium Stearate | 3.0 |
| | **Final Tablet weight (mg)** | 1154.9 |

Manufacturing Instructions for Sustained Release Granulation:
1. Mix Paracetamol, HPMC 2208, PVP, HPMC 2910 and Pregel Starch in a high shear granulator.
2. Add purified water while mixing.
3. Continue mixing until suitable granulation end point is attained.
4. Dry the granulation to the target % LOD.
5. Milled dry granulation using Co-mill equipped with suitable size screen

Manufacturing Instructions for Final Blend:
1. Add sustained release blend to a suitable low shear blender.
2. Add Kollidon-SR and mix.
3. Add lubricant, magnesium stearate and mix.

Blend is now ready for compression on a suitable size rotary tablet press.

### EXAMPLE 4 (not according to the invention)

In another example, a single layer sustained release acetaminophen tablet is prepared using the following ingredients:

### EXAMPLE - 4 (Single Layer Enteric Coated Tablet)

| **No.** | **INGREDIENT** | **mg per tablet** |
|---|---|---|
| | Intragranular Components: | |
| 1 | Paracetamol fine | 900.0 |
| 2 | Hypromellose (HPMC) 2208 4000 cP | 50.0 |
| 3 | Povidone K25 | 20.0 |
| 4 | Pregel starch fine | 10.0 |
| 5 | Hypromellose (HPMC) 2910 15 cP | 16.0 |
| | Extragranular Components: | |
| 6 | Paracetamol (DC90) | 111.2 |
| 7 | Magnesium Stearate | 3.0 |

| | **Enteric Coat** | |
|---|---|---|
| 8 | Acryl-EZ | 44.4 |
| 9 | Triethyl Citrate | 4.4 |
| | **Final Tablet weight (mg)** | 1159.0 |

Manufacturing Instructions for Sustained Release Granulation:
1. Mix Paracetamol, HPMC 2208, PVP, HPMC 2910 and Pregel Starch in a high shear granulator.
2. Add purified water while mixing.
3. Continue mixing until suitable granulation end point is attained.
4. Dry the granulation to the target % LOD.
5. Milled dry granulation using Co-mill equipped with suitable size screen
6. Add DC 90 (paracetamol) and mix for 5 minutes
7. Add lubricant, magnesium stearate and mix.
8. Blend is now ready for compression
9. Compressed single layer tablets on a suitable size rotary tablet press.
10. Tablets are then coated in a suitable size coating pan using Acryl-EZ as an enteric coat.

### EXAMPLE 5 (not according to the invention)

In this example, three different Bilayer sustained release paracetamol tablets are prepared using the following ingredients:

### EXAMPLE - 5 (Bi-Layer Tablet)

| **No.** | **INGREDIENT** | **5A** | **5B** | **5C** |
|---|---|---|---|---|
| **A** | **Sustained Release Layer (SR Layer)** | **mg per tablet** | | |
| 1 | Paracetamol fine | 800.0 | 900.0 | 800.0 |
| 2 | Hypromellose (HPMC) 2208 4000 cP | 48.0 | 27.0 | 24.0 |
| 3 | Povidone K25 | -- | -- | -- |
| 4 | Pregel starch fine | -- | -- | -- |
| 5 | Hypromellose (HPMC) 2910 15 cP | -- | -- | -- |
| 6 | Magnesium Stearate | 3.0 | 3.0 | 3.0 |
| | | | | |

| **B** | **Immediate Release Layer (IR Layer)** | | | |
|---|---|---|---|---|
| 7 | Paracetamol (DC90) | 222.4 | 111.2 | 222.4 |

| **Bi-Layer Tablet** | | | | |
|---|---|---|---|---|
| **A** | Sustained Release Layer (SR Layer) | 851.0 | 930.0 | 827.0 |
| **B** | Immediate Release Layer (IR Layer) | 222.4 | 111.2 | 222.4 |
| | **Final Tablet weight (mg)** | 1073.4 | 1041.2 | 1049.4 |

Manufacturing Instructions for Sustained Release Layer:
1. Mix Paracetamol and HPMC 2208, in a high shear granulator.
2. Add purified water while mixing.
3. Continue mixing until suitable granulation end point is attained.
4. Dry the granulation to the target % LOD.
5. Milled dry granulation using Co-mill equipped with suitable size screen
6. Add lubricant, magnesium stearate and mix.
7. Blend is now ready for compression

Manufacturing Instructions for Immediate release Layer:
DC 90 paracetamol, a commercially available blend (paracetamol is 90% w/w) is used in the immediate release layer, and the tablets are compressed on a suitable size bi-layer rotary tablet press.

The biorelevant dissolution profiles of the formulations listed in the Example 5 above, are shown in Figure 1b. It is recognized that these formulations are outside the scope of the present invention. They are provided as a demonstration of the principles of the IVMS formulation selection and prediction process.

### EXAMPLE 6

In this example, two different Bi-layer Sustained Release paracetamol tablets are prepared using the following ingredients:

### EXAMPLE - 6

| **No.** | **INGREDIENT** | **6A** | **6B** |
|---|---|---|---|
| **A** | **Sustained Release Layer (SR Layer)** | **mg per tablet** | |
| 1 | Paracetamol fine | 800.0 | 900.0 |
| 2 | Hypromellose (HPMC) 2208 4000 cP | 32.0 | 36.0 |
| 3 | Povidone K25 | 20.0 | 22.5 |
| 4 | Pregel starch fine | 10.0 | 11.3 |
| 5 | Hypromellose (HPMC) 2910 15 cP | 16.0 | 18 |
| 6 | Magnesium Stearate | 3.0 | 3.4 |

| **B** | **Immediate Release Layer (IR Layer)** | | |
|---|---|---|---|
| 7 | Paracetamol (DC90) | 222.4 | 111.2 |

| **Bi-Layer Tablet** | | | |
|---|---|---|---|
| **A** | Sustained Release Layer (SR Layer) | 881.0 | 991.1 |
| **B** | Immediate Release Layer (IR Layer) | 222.4 | 111.2 |
| | **Final Tablet weight (mg)** | 1103.4 | 1102.3 |

Manufacturing Instructions for Sustained Release Layer:
1. Mix Paracetamol, HPMC 2208, PVP, HPMC 2910 and Pregel Starch in a high shear granulator.
2. Add purified water while mixing.
3. Continue mixing until suitable granulation end point is attained.
4. Dry the granulation to the target % LOD.
5. Milled dry granulation using Co-mill equipped with suitable size screen
6. Add lubricant, magnesium stearate and mix.
7. Blend is now ready for compression

Manufacturing Instructions for Immediate release Layer:
1. DC 90 paracetamol is used as an immediate release layer

The biorelevant dissolution profile of the formulations listed in the Example 6 above, are shown in Figure 1b.

### EXAMPLE 7 (not according to the invention)

Herein disclosed is an extended release paracetamol tablet wherein the dissolution rate is controlled by the Hypromellose component. A current available supplier of HPMC is Shin-Etsu with the grade being identified as 90SH-4000SR having an average viscosity of 4000cps. In order to identify the critical parameters of HPMC that control dissolution and compaction behavior, four different grades of HPMCs from two suppliers were evaluated - 90SH-4000 & 90SH-4000SR (Shin-Etsu Chemical Co., Ltd.) and K4M & K4MCR (Colorcon). All four grades had the same HPMC viscosity range, 3600-5200cps as specified in the USP. The main difference amongst the two grades within the same supplier was in the particle size with the SR and CR grade generally being the preferred ones for controlled release formulation due to their small and narrow particle size distribution. In addition, lots of K4M and K4MCR at the higher end of the viscosity specification range were also used to determine the effect of viscosity on dissolution.

An Ishikawa diagram was used to identify the HPMC characteristics that can potentially impact dissolution and compaction. Pre-formulation studies were done to study these properties. Tablets were made with the various HPMC grades using the formulation of EXAMPLE 2 and processes thereof. Both a FBRM profile and power curve were recorded during the granulation process. Tablets were compressed to the same target hardness and tableting parameters recorded. Dissolution studies were conducted using the method described herein in a USP Apparatus II in pH 7.4 buffer.

All the formulations made with the various HPMCs met the required dissolution specification. Based on the f₁/f₂ criteria, however, differences were noted among the HPMC grades depending on the Tg, viscosity and specific surface area, which were confirmed to be the critical quality attributes for HPMC. 90SH-4000 with the highest Tg and largest surface area gave a higher dissolution compared to other grades. All grades/lots of HPMC showed similar compaction and flow behavior compared to the 90SH-4000SR grade.

### EXAMPLE 8

### IVMS method:

*In vivo* modeling and simulation (IVMS) is a physiologically-based drug ADME, PK/PD modeling and simulation tool. IVMS provides a platform for drug absorption, distribution, metabolism and excretion (ADME) in virtual populations. IVMS can effectively guide and assemble the development of new target formulations and optimum line extensions of drug products including OTC medicines.

In the present sustained release formulations, as the bio-dissolution method was developed and formulations were tested, the dissolution data was linked to the IVMS work and processed according to the IVMS model. IVMS methods and principles were published in 2002-2008. With use of these methods and principles it was unexpectedly discovered that with the bi-layered formulation of Example 1 and the single layer formulation of Example 2, a 4µg/ml paracetamol plasma level could be reached and sustained for the necessary therapeutic duration. The formulations of Examples 2-4 are not according to the invention.

**Table 1**

| **Predicted (IVMS) vs. Observed Therapeutic Effective Time (TET) Values** | | |
|---|---|---|
| | **Single Dose** | **Clinical Study** |
| **Formulation Candidates (Recommended Rank before Study Start)** | **Predicted Time ≥ 4µg/ml** | **Observed Time ≥ 4µg/ml (M ± SD)** |
| Example 1 | 8.8 | 8.1 (6.1 ∼ 10.1) |
| Example 2 | 8.7 | 7.3 (6.3 ∼ 9.0) |
| Example 3 | 8.2 | 7.5 (5.0 ∼ 10.0) |
| Example 4 | N/A | 7.1 (3.5 ∼ 10.6) |

### Pharmacokinetic (PK) Variables:

The PK characteristics of Example Formulations 1 to 4 herein were assessed by a single dose clinical study in semi-fed state. Surprisingly, it was found that the formulation of Example 1 gave plasma concentrations above 4µg/ml for the longest period of time (8.1 hours). This was similar to the time-period of two doses of a standard paracetamol formulation (8.3 hours). Example 1 Formulation maintained plasma paracetamol concentrations higher than 3µg/ml up to the 11^{th} hour post-dose. The Example 1 Formulation also had the highest mean value of AUC_{(0-12 hours)} (75.2 µg*h/ml). The T*ₘₐₓ* value for the Example 1 Formulation (4.5 hours) was among the highest of the formulations. This is surprising as it was expected that a bi-layer tablet containing 100 mg APAP in the immediate layer portion would have a lower Tₘₐₓ than the single layer formulation of Example 3 which contained no immediate release paracetamol (Tₘₐₓ =4.0 hours). The formulation of Example 1 also demonstrated the highest T ≥ 5µg/ml among the 4 formulations as shown below. The T ≥ 3µg/ml for the formulation of Example 1 (10.3 hours) was similar to the other 3 candidates.

**Table 3**

| **Summary statistics for time of plasma paracetamol concentration equal or greater than 5, 4, 3 and 2 µg/mL** | | | | | | |
|---|---|---|---|---|---|---|
| Time - T (hours) | Statistics | Example 1 1 g x 2 at 0 hr | Example 2 1 g x 2 at 0 hr | Example 3 1 g x 2 at 0 hr | Example 4 1 g x 2 at 0 hr | Conventional IR Tablet 0.5g x 2 at 0 and 6 hrs |
| **T ≥ 5µg/mL** | **Mean (hours)** | **6.3** | **5.5** | **5.4** | **5.2** | **6.6** |
| | Min (hours) | 4.0 | 3.0 | 2.0 | 1.0 | 4.5 |
| | Max (hours) | 10.0 | 9.0 | 11.0 | 10.0 | 11.0 |
| | CV (%) | 31 | 32 | 43 | 50 | 26 |
| **T ≥ 4µg/mL** | **Mean (hours)** | **8.1** | **7.3** | **7.5** | **7.1** | **8.3** |
| | Min (hours) | 5.0 | 5.0 | 3.0 | 3.0 | 5.5 |
| | Max (hours) | 12.0 | 10.5 | 14.0 | 17.5 | 12.0 |
| | CV (%) | 25 | 23 | 34 | 50 | 22 |
| **T ≥ 3µg/mL** | **Mean (hours)** | **10.3** | **10.3** | **10.6** | **10.1** | **10.1** |
| | Min (hours) | 7.0 | 7.0 | 6.0 | 5.5 | 7.0 |
| | Max (hours) | 14.0 | 16.0 | 17.0 | 17.5 | 13.0 |
| | CV (%) | 20 | 25 | 26 | 40 | 18 |

### PK Data from Steady State Clinical Study

The formulation of Example 1 was evaluated to determine bioequivalence at steady state (final 24 hours of dosing) between 2000mg paracetamol of Example 1 given twice a day, 1330mg dose of an 8-hour extended release paracetamol formulation, Panadol® Extend given 3 times a day and 1000mg of a conventional immediate release paracetamol formulation, Panadol®, given 4 times a day for 3 days. At steady state, 2000mg of Example 1 Formulation (2000mg paracetamol twice a day) was bioequivalent to both an 8-hour extended release paracetamol formulation, Panadol® Extend (1330mg paracetamol 3 times a day) and a conventional immediate release paracetamol formulation, Panadol® (1000mg paracetamol 4 times a day) with regards to paracetamol C*ₘₐₓ*, AUC₀₋ₜ and AUC_{0-inf}. Over a 24 hr period at steady state the mean time period where the plasma paracetamol was greater than or equal to 4µg/ml for Example 1 was significantly longer (1.5 hours) than the conventional immediate release formulation, Panadol® dosed four times a day. This is clinically relevant as well as statistically significant (P=0.0046).

### PK Data from Pivotal PK Studies (Fasted & Fed) Vs Conventional Immediate Release Formulation

The PK characteristics of the bi-layer formulation of Example 1 were assessed in a single dose study in order to determine absorption and food effect characteristics of the formulation in a fed and fasted state. The formulation of example 1 was given as a single 2000mg paracetamol dose (1000mg x 2 tablets). The conventional immediate release formulation Pandaol ® was given as a single 1000mg dose (500mg x 2 tablets). 2000mg of the Example 1 Formulation was well absorbed in both fasted and fed states with more than 90% relative bioavailability as compared to a conventional immediate release formulation, Panadol®. Time at or above the therapeutic level (≥ 4µg/ml) from 2000mg of Example 1 formulation was approximately double that of one dose (1000mg) of standard Panadol®. Surprisingly food had a significant effect on the peak exposure of paracetamol by increasing the C*ₘₐₓ* of 2000mg Example 1 formulation and decreasing the C*ₘₐₓ* of Panadol®. Food caused a significant decrease in overall extent of paracetamol absorption (AUC_{0-inf}) for Panadol® but had less impact for the Example 1 Formulation.

### Pharmacodynamics

There is no well established PK-PD link for paracetamol. Conventional thinking suggests that in order for paracetamol to be effective, paracetamol must be present in the blood plasma at a concentration of at least 3µg/ml-4µg/ml. It is believed that time above at least 3µg/ml, and more importantly time above 4µg/ml, may be clinically significant. Time above 5µg may also be clinically significant.

Thus, one aspect of the present invention is the time that paracetamol must be present in the blood at a concentration of at least 3µg/ml, such as for about a 10 hour window.

Another aspect of the present invention is the time that paracetamol must be present in the blood at a concentration of at least 4µg/ml, such as for about an 8 hour window. Another aspect of the present invention is the time that paracetamol must be present in the blood at a concentration of at least 5µg/ml, such as for about a 6 hour window, when dosed at 2000mg paracetamol twice daily, and as compared to the conventional immediate release formulation when taken as a 1000mg dose four times per day.

Another aspect of the invention is the plasma C*ₘₐₓ* value obtained by the formulations of the present invention. Even though the formulations of the present invention were given at a higher dose of paracetamol (2000mg) than other conventional formulations, the C*ₘₐₓ* in the fasted state is lower than a conventional immediate release formulation dosed at half that amount (1000mg). As a result of a lower C*ₘₐₓ*, additional adverse effects would not be expected to be observed with the formulations of the present invention as compared to a conventional immediate release formulation.

The minimum time that a formulation should be at or above the desired plasma levels is also an aspect of this invention. As shown in Table 3 above, one aspect of the invention is a mean plasma concentration of at least 5µg/ml which should be maintained for at least about 6 hours; or a mean plasma concentration of at least 4ug/ml which should be maintained for at least about 8.0 hours; or mean plasma concentration of at least about 3ug/ml for at least about 10 hours. The mean plasma concentration should be maintained for these time periods longer than a standard immediate release formulation.

In another embodiment of the invention, the extent of absorption of the paracetamol should be equivalent to a conventional immediate release paracetamol. It is also desired that the therapeutically active drug plasma concentration of paracetamol should be attained rapidly by the immediate release phase of the paracetamol.

Another aspect of the invention is the slope of the decline in plasma level of APAP. In a repeat dose clinical study, the elimination rate constant (Kₑₗ) for the formulation of the present invention was 25% lower than that for the conventional immediate release formulation. This is related to a slower controlled release rate of paracetamol from the tablets of the formulation, such as those exemplified by Example 1, as compared to a conventional immediate release formulation. The elimination rate constants of Example 1 tablets and the tablets of an 8-hr extended release formulation, Panadol® Extend, were also found to be comparable (0.26 hr⁻¹ and 0.27 hr⁻¹, respectively).

A biorelevant dissolution process was developed to reflect in vivo drug release or absorption. Dissolution of various commercially available sustained release products was assessed in different dissolution media, at different pH and different rotations per minute. The data was correlated with known in vivo data for the same formulation. The best correlation was obtained with a USP Apparatus II in 900 ml of 40mM phosphate buffer, at pH 7.4 and at 75 RPM. This fluid was used for all the tested formulations.

Using a biorelevant dissolution model such as that described herein, Figures 1a & 1b demonstrate the dissolution characteristics of the example formulations and commercially available immediate release paracetamol formulation, Panadol®, and an 8 hour extended release formulation, Panadol® Extend.

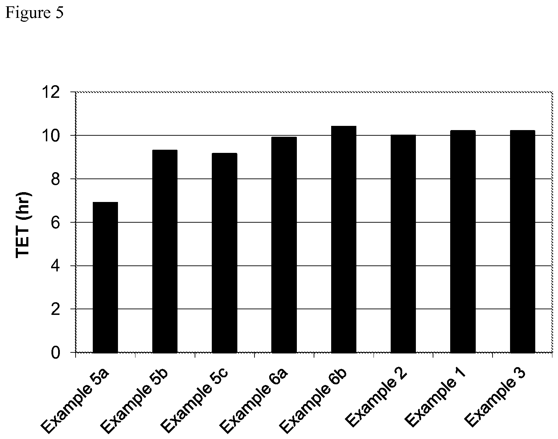

Figure 5 demonstrates the Therapeutic Effect Time (TET) of the various example formulations.

### References

1. J.T. DiPiro, R.L. Talbert, G.C. Yee, et al., Pharmacotherapy: A Pathophysiologic Approach, 7e.
2. C. Dollery. Therapeutic drugs, vol 1; 2nd ed. Churchill Livingston Publications: 1999. p A19-21.
3. M Hossain, J.W. Ayres, Pharmacokinetics and pharmacodynamics in the design of controlled-release beads with acetaminophen as model drug. J Pharm Sci 1992; 81: 444-448.
4. D.J. Liu, et al., Simulating/predicting in vivo absorption profile. AAPS Workshop on Computer Simulation and Its Role in Drug Development Research. 2002
5. D.J. Liu, Profiling Drug Absorption and Metabolism by In Situ Gastrointestinal Perfusion Studies to Predict in vivo Performance of Oral Drugs. American Association of Pharmaceutical Scientists (AAPS) Annual Meeting, 2008
6. D.J. Liu, G. Davies, et al., New IVMS approach to develop novel oral drug formulation, American Association of Pharmaceutical Scientists (AAPS) Annual Meeting, 2010
7. J.C. Nielsen, P. Bjerring, L. Arendt-Nielsen. A comparison of the hypoalgesic effect of paracetamol in slow-release and plain tablets on laser-induced pain. Br J Clin Pharmacol 1991;31: 267-270.
8. D.J. Liu, Apply In Vivo Modeling and Simulation to Identify the Minimum Therapeutic/Effective Doses (MTD/MED) of Paracetamol for Pain Relief. 8th World Congress World Institute of Pain Management Annual Meeting, 2012

## Claims

1. A sustained release formulation for oral administration containing 2000mg of paracetamol per unit dose administered as two bilayer tablets, each tablet containing 1000mg of paracetamol present in a sustained release phase and in an immediate release phase, in which the ratio of the paracetamol in the sustained release phase to the immediate release phase is 80-90% to 10-20%, and wherein the sustained release phase comprises 4% to 6% by weight of at least one high viscosity hypromellose, 0.5% to 3% by weight of at least one binding agent which is povidone, from 1% to 2% by weight of a low viscosity hypromellose, 0.5% to 3% by weight of at least one modified starch, and wherein the ratio of high-viscosity hypromellose to low viscosity hypromellose is 3.3 to 0.85, wherein when ingested by a human, the formulation reduces the maximum attained plasma-paracetamol concentration by at least about 4.5% at steady state (relative to rapid-release paracetamol formulations'), and increases the time to reach a maximum paracetamol-plasma concentration (T*ₘₐₓ*) by at least about 140% at steady state (relative to rapid-release paracetamol formulations'), while having an insignificant effect on area under the plasma-paracetamol concentration time curve AUC₍₀₋₂₄₎; mean AUC₍₀₋₂₄₎ of about 165 µg*h/ml for sustained release paracetamol at steady state (2000mg dosed every 12 hours) versus a mean AUC₍₀₋₂₄₎ of about 168 µg*h/ml for 1000mg immediate release at steady state (dosed every 6 hours) and wherein the formulation is repeatedly administered (steady state).

2. A sustained release formulation according to claim 1, wherein the ratio of high-viscosity hypromellose to low viscosity hypromellose is about 3:1.

3. A sustained release formulation according to claim 1, in which the ratio of paracetamol in the sustained release phase to the immediate release phase is 90:10.

4. A sustained release formulation as claimed in any one of claims 1-3 for oral administration providing a therapeutically effective plasma paracetamol concentration over a 12 hour period for use in the treatment of pain in a human in need thereof wherein the formulation has the following *in vitro* bio-dissolution profile of the dissolution release ranges at various time points (as determined by USP Type II apparatus, rotating paddle, with 900 ml of Phosphate buffer at pH 7.4, 37 °C set at rotating speed of 75rpm) of:
• 2 to 15% released at 15 minutes;
• 4 to 22% released at 30 minutes;
• 10 to 40% released at 60 minutes;
• 22 to 62% released at 180 minutes;
• 50 to 88% released at 360 minutes;
• >90% released after 720 minutes.

## Patentansprüche

1. Formulierung mit verzögerter Freisetzung zur oralen Verabreichung, die 2000 mg Paracetamol pro Einheitsdosis enthält, die als zwei Zweischichttabletten verabreicht wird, worin jede Tablette 1000 mg Paracetamol in einer Phase mit verzögerter Freisetzung und in einer Phase mit sofortiger Freisetzung enthält, in der das Verhältnis von Paracetamol in der Phase mit verzögerter Freisetzung zur Phase mit sofortiger Freisetzung 80-90% zu 10-20% beträgt, und worin die Phase mit verzögerter Freisetzung 4 Gew.-% bis 6 Gew.-% mindestens einer hochviskosen Hypromellose, 0,5 Gew.-% bis 3 Gew.-% mindestens eines Bindemittels, das Povidon ist, 1 Gew.-% bis 2 Gew.-% einer niederviskosen Hypromellose und 0,5 Gew.-% bis 3 Gew.-% mindestens einer modifizierten Stärke umfasst, und worin das Verhältnis der hochviskosen Hypromellose zur niederviskosen Hypromellose 3,3 bis 0,85 beträgt, worin die Formulierung, wenn sie von einem Menschen aufgenommen wird, die maximal erreichte Paracetamol-Plasmakonzentration um mindestens etwa 4,5% im stationären Zustand reduziert (relativ zu der von schnell freisetzenden Paracetaol-Formulierungen) und die Zeit bis zum Erreichen einer maximalen Paracetamol-Plasmakonzentration (Tₘₐₓ) um mindestens etwa 140% im stationären Zustand erhöht wird (relativ zu der von schnell freisetzenden Paracetamol-Formulierungen), während sie einen unbedeutenden Effekt auf die Fläche unter der Paracetamol-Plasmakonzentrations-Zeit-Kurve AUC₍₀₋₂₄₎ hat; mittlere AUC₍₀₋₂₄₎ von etwa 165 µg*h/ml für Paracetamol mit verzögerter Freisetzung in stationärem Zustand (2000 mg alle 12 Stunden dosiert) gegenüber einer mittleren AUC₍₀₋₂₄₎ von etwa 168 µg*h/ml für die sofortige Freisetzung von 1000 mg im stationären Zustand (Dosierung alle 6 Stunden), und worin die Formulierung wiederholt verabreicht wird (im stationären Zustand).

2. Formulierung mit verzögerter Freisetzung gemäß Anspruch 1, worin das Verhältnis von hochviskoser Hypromellose zu niederviskoser Hypromellose ungefähr 3:1 beträgt.

3. Formulierung mit verzögerter Freisetzung gemäß Anspruch 1, in der das Verhältnis von Paracetamol in der Phase mit verzögerter Freisetzung zur Phase mit sofortiger Freisetzung 90:10 beträgt.

4. Formulierung mit verzögerter Freisetzung wie in den Ansprüchen 1-3 beansprucht zur oralen Verabreichung, die eine therapeutisch wirksame Paracetamol-Plasmakonzentration über einen Zeitraum von 12 Stunden bewirkt, zur Verwendung bei der Behandlung von Schmerz bei einem Menschen, der diese benötigt, worin die Formulierung das folgende *in vitro-*Bioauflösungsprofil an Auflösungsfreisetzungsbereichen zu verschiedenen Zeitpunkten aufweist (bestimmt mittels eines USP Typ II Apparats, Drehflügel, mit 900 ml Phosphatpuffer bei pH 7,4, bei 37°C auf eine Drehgeschwindigkeit von 75 U/min eingestellt):
• 2 bis 15% freigesetzt bei 15 Minuten;
• 4 bis 22% freigesetzt bei 30 Minuten;
• 10 bis 40% freigesetzt bei 60 Minuten;
• 22 bis 62% freigesetzt bei 180 Minuten;
• 50 bis 88% freigesetzt bei 360 Minuten;
• >90% freigesetzt bei 720 Minuten.

## Revendications

1. Formulation à libération prolongée pour administration orale contenant 2000 mg de paracétamol par dose unitaire administrée sous la forme de comprimés bicouches, chaque comprimé contenant 1000 mg de paracétamol présent dans une phase à libération prolongée et dans une phase à libération immédiate, dans laquelle la proportion du paracétamol dans la phase à libération prolongée par rapport à la phase à libération immédiate est de 80-90 % à 10-20 %, et dans laquelle la phase à libération prolongée comprend 4 % à 6 % en poids d'au moins une hypromellose de viscosité élevée, 0,5 % à 3 % en poids d'au moins un agent de liaison qui est la povidone, de 1 % à 2 % en poids d'une hypromellose de viscosité basse, 0,5 % à 3 % en poids d'au moins un amidon modifié, et dans laquelle le rapport entre l'hypromellose de viscosité élevée et l'hypromellose de viscosité basse est de 3,3 à 0,85, dans laquelle quand elle est ingérée par un humain, la formulation réduit la concentration plasmatique en paracétamol maximale atteinte d'au moins environ 4,5 % à l'état d'équilibre (par rapport aux formulations de paracétamol à libération rapide), et augmente la durée pour atteindre une concentration plasmatique maximale en paracétamol (T*ₘₐₓ*) d'au moins environ 140 % à l'état d'équilibre (par rapport aux formulations de paracétamol à libération rapide), tout en ayant un effet insignifiant sur la surface sous la courbe de concentration plasmatique en paracétamol-temps SSC₍₀₋₂₄₎ ; SSC₍₀₋₂₄₎ moyenne d'environ 165 µg*h/ml pour le paracétamol à libération prolongée à l'état d'équilibre (2000 mg dosés toutes les 12 heures) par rapport à une SSC₍₀₋₂₄₎ moyenne d'environ 168 µg*h/ml pour 1000 mg de libération immédiate à l'état d'équilibre (dosés toutes les 6 heures) et dans laquelle la formulation est administrée à plusieurs reprises (état d'équilibre).

2. Formulation à libération prolongée selon la revendication 1, dans laquelle le rapport entre l'hypromellose de viscosité élevée et l'hypromellose de viscosité basse est d'environ 3 : 1.

3. Formulation à libération prolongée selon la revendication 1, dans laquelle la proportion de paracétamol dans la phase à libération prolongée par rapport à la phase à libération immédiate est de 90 : 10.

4. Formulation à libération prolongée selon l'une quelconque des revendications 1 à 3 pour une administration orale fournissant une concentration plasmatique en paracétamol thérapeutiquement efficace sur une période de 12 heures pour une utilisation dans le traitement de la douleur chez un humain en ayant besoin dans laquelle la formulation présente le profil de bio-dissolution *in vitro* suivant des plages de libération par dissolution à divers moments temporels (tel que déterminé par un appareil USP de type II, aube rotative, avec 900 ml de tampon au phosphate à pH 7,4, 37 °C réglé à une vitesse de rotation de 75 tours/minute) de :
• 2 à 15 % libérés à 15 minutes ;
• 4 à 22 % libérés à 30 minutes ;
• 10 à 40 % libérés à 60 minutes ;
• 22 à 62 % libérés à 180 minutes ;
• 50 à 88 % libérés à 360 minutes ;
• > 90 % libérés après 720 minutes.
